# EUROPEAN PATENT SPECIFICATION

(11) **EP 4 398 828 B1**
(45) Date of publication and mention of the grant of the patent: **02.12.2026**
(21) Application number: 23738892.1
(22) Date of filing: 27.06.2023
(51) Int. Cl.: A61B 18/14, A45C 13/10, A61B 17/32

(54) **SURGICAL INSTRUMENT WITH PREDETERMINED SEPARATION FEATURE FOR WASTE STREAM UTILIZATION AND RELATED METHODS**
CHIRURGISCHES INSTRUMENT MIT VORBESTIMMTER TRENNFUNKTION ZUR VERWENDUNG VON ABFALLSTRÖMEN UND ZUGEHÖRIGE VERFAHREN
INSTRUMENT CHIRURGICAL DOTÉ D'UNE FONCTION DE SÉPARATION PRÉDÉTERMINÉE POUR L'UTILISATION DU FLUX RÉSIDUEL ET PROCÉDÉS CONNEXES

(30) Priority: 30.06.2022 US 202217854050
(43) Date of publication of application: 17.07.2024
(73) Proprietor: Cilag GmbH International, 6300 Zug (CH)
(72) Inventor: SHELTON, IV, Frederick E., Cincinnati, Ohio 45242 (US); ARONHALT, Jacqueline C., Cincinnati, Ohio 45242 (US); ADAMS, Shane R., Cincinnati, Ohio 45242 (US); ARONHALT, Taylor W., Cincinnati, Ohio 45242 (US); CARRANZA, Robert N., Cincinnati, Ohio 45242 (US); CHOW, Aaron J., Cincinnati, Ohio 45242 (US); COWPERTHWAIT, Matthew D., Cincinnati, Ohio 45242 (US); LUCAS, Guion Y., Cincinnati, Ohio 45242 (US); ROSS, Nicholas J., Cincinnati, Ohio 45242 (US); SCOGGINS, Patrick J., Cincinnati, Ohio 45242 (US)
(74) Representative: Carpmaels & Ransford LLP
(86) International application number: PCT/IB2023/056602
(87) International publication number: WO 2024/003722

(56) References cited:
- CN-A- 111 920 164
- US-A1- 2009 259 793
- US-A1- 2013 103 023
- US-A1- 2015 265 307
- US-A1- 2017 119 427
- US-A1- 2017 202 591
- US-A1- 2021 346 086
- ANONYMOUS: "pcb design - Detachable "debug" portion of PCB? - Electrical Engineering Stack Exchange", 15 February 2018 (2018-02-15), XP093104291, Retrieved from the Internet <URL:https://electronics.stackexchange.com/questions/356423/detachable-debug-portion-of-pcb> [retrieved on 20231122]

## Description

### BACKGROUND

A variety of ultrasonic surgical instruments include an end effector having a blade element that vibrates at ultrasonic frequencies to cut and/or seal tissue (e.g., by denaturing proteins in tissue cells). These instruments include one or more piezoelectric elements that convert electrical power into ultrasonic vibrations, which are communicated along an acoustic waveguide to the blade element. Examples of ultrasonic surgical instruments and related concepts are disclosed in U.S. Pub. No. 2006/0079874, entitled "Tissue Pad for Use with an Ultrasonic Surgical Instrument," published April 13, 2006; U.S. Pub. No. 2007/0191713, entitled "Ultrasonic Device for Cutting and Coagulating," published August 16, 2007; and U.S. Pub. No. 2008/0200940, entitled "Ultrasonic Device for Cutting and Coagulating," published August 21, 2008.

Some instruments are operable to seal tissue by applying radiofrequency (RF) electrosurgical energy to the tissue. Examples of such devices and related concepts are disclosed in U.S. Pat. No. 7,354,440, entitled "Electrosurgical Instrument and Method of Use," issued April 8, 2008; and U.S. Pat. No. 7,381,209, entitled "Electrosurgical Instrument," issued June 3, 2008.

Some instruments are capable of applying both ultrasonic energy and RF electrosurgical energy to tissue. Examples of such instruments are described in U.S. Patent No. 9,949,785, entitled "Ultrasonic Surgical Instrument with Electrosurgical Feature," issued April 24, 2018; U.S. Patent No. 8,663,220, entitled "Ultrasonic Electrosurgical Instruments," issued March 4, 2014; U.S. Patent No. 10,835,307, entitled "Modular Battery Powered Handheld Surgical Instrument Containing Elongated Multi-Layered Shaft," issued November 17, 2020; and U.S. Patent No. 11,229,471, entitled "Modular Battery Powered Handheld Surgical Instrument with Selective Application of Energy Based on Tissue Characterization," issued January 25, 2022.

In some scenarios, it may be preferable to have surgical instruments grasped and manipulated directly by the hand or hands of one or more human operators. In addition, or as an alternative, it may be preferable to have surgical instruments controlled via a robotic surgical system. Examples of robotic surgical systems and associated instrumentation are disclosed in U.S. Pat. No. 10,624,709, entitled "Robotic Surgical Tool with Manual Release Lever," published on May 2, 2019; U.S. Pat. No. 9,314,308, entitled "Robotic Ultrasonic Surgical Device With Articulating End Effector," issued on April 19, 2016; U.S. Pat. No. 9,125,662, entitled "Multi-Axis Articulating and Rotating Surgical Tools," issued September 8, 2015; U.S. Pat. No. 8,820,605, entitled "Robotically-Controlled Surgical Instruments," issued Sep. 2, 2014; U.S. Pub. No. 2019/0201077, entitled "Interruption of Energy Due to Inadvertent Capacitive Coupling," published July 4, 2019; U.S. Pub. No. 2012/0292367, entitled "Robotically-Controlled End Effector," published on November 11, 2012; and U.S. Pat. App. No. 16/556,661, entitled "Ultrasonic Surgical Instrument with a Multi-Planar Articulating Shaft Assembly," filed on August 30, 2019.

Such instruments and robotic surgical systems may be further be incorporated into a surgical system for performing procedures in a surgical environment, such as surgical operating theaters or rooms in a healthcare facility. A sterile field is typically created around the patient and may include properly attired, scrubbed healthcare professions as well as desired furniture and/or fixtures. Examples of such surgical systems and associated features are disclosed in U.S. Pat. Pub. No. 2019/0201046, entitled "Method for Controlling Smart Energy Devices," published on July 4, 2019; U.S. Pat. Pub. No. 2019/0201080, entitled "Ultrasonic Energy Device Which Varies Pressure Applied by Clamp Arm to Provide Threshold Control Pressure at a Cut Progression Location," published on July 4, 2019; U.S. Pat. Pub. No. 2019/0201091, entitled "Radio Frequency Energy Device for Delivering Combined Electrical Signals," published July 4, 2019; U.S. Pat. Pub. No. 2019/0274717, entitled "Methods for Controlling Temperature in Ultrasonic Device," published September 12, 2019; and U.S. Pat. Pub. No. 2019/0207857, entitled "Surgical Network Determination of Prioritization of Communication, Interaction, or Processing Based on System or Device Needs," published July 4, 2019. US 2017/0202591 A1 discloses a modular battery powered handheld surgical instrument with selective application of energy based on tissue characterization. US 2015/0265307 A1 discloses a battery-powered hand-held ultrasonic surgical cautery cutting device. US 2013/0103023 A1 discloses a litz wire battery powered device. US 2017/0119427 A1 discloses a cordless hand-held ultrasonic cautery device. CN 111920164A discloses a luggage case with magnetic locking mechanism.

While several surgical instruments and systems have been made and used, it is believed that no one prior to the inventors has made or used the invention described in the appended claims.

### SUMMARY OF THE INVENTION

The present invention provides a surgical instrument as recited in claim 1 and a method of disassembling the surgical instrument according to claim 14. Optional features are recited in the dependent claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a block diagram of an example a computer-implemented interactive surgical system;
FIG. 2 depicts a top schematic view of an example of a surgical system for performing a surgical procedure in an operating room of a healthcare facility;
FIG. 3 depicts a side schematic view of an example of a surgical hub of the surgical system of FIG. 2;
FIG. 4 depicts a perspective view of a combination generator module with bipolar, ultrasonic, and monopolar contacts of the surgical system of FIG. 2;
FIG. 5 depicts a side schematic view of an exemplary generator and various examples of surgical instruments for use with the surgical system of FIG. 2;
FIG. 6A depicts a side view of a surgical instrument including a plurality of selectively removeable shrouds attached by a plurality of magnetic members in a connected state;
FIG. 6B depicts a partially exploded, side view of the surgical instrument of FIG. 6A with the shrouds separated from one another in a disconnected state;
FIG. 7A depicts a side view of a surgical instrument including a magnetic lock assembly in a locked state and a plurality of selectively removeable shrouds retained by the magnetic lock assembly in a connected state;
FIG. 7B depicts a partially exploded side view of the surgical instrument of FIG. 7A with the magnetic lock assembly in an unlocked state and the shrouds separated from one another in a disconnected state;
FIG. 8A depicts a perspective view of a surgical instrument including a shroud attached by push-pins in a connected state and in electrical communication with a generator;
FIG. 8B depicts a perspective view of the surgical instrument of FIG. 8A with the shrouds separated from one another in a disconnected state;
FIG. 9 depicts an enlarged perspective view of a push-pin being removed from the shroud of the surgical instrument of FIG. 8A;
FIG. 10 depicts a side view of another push-pin for use with surgical instrument of FIG.8A;
FIG. 11A depicts a perspective view of a portion of a surgical instrument with a shroud removed exposing a main circuit board with a plurality of pluggable sub-boards in an installed position;
FIG. 11B depicts a perspective view of the portion of the surgical instrument of FIG. 11A with the shroud removed and the plurality of pluggable sub-boards in an uninstalled position;
FIG. 12 depicts a perspective view of a portion of a surgical instrument with a shroud removed exposing a main circuit board including a frangible separator in an intact state;
FIG. 13 depicts a perspective view of the main circuit board of the surgical instrument of FIG. 12 with the main circuit board in a separated state;
FIG. 14A depicts a sectional view of a surgical instrument including a latch in a latched position and a memory member in an operable state;
FIG. 14B depicts a sectional view of the surgical instrument of FIG. 14A including the latch in an unlatched position after passing over the memory member rendering the memory member in an inoperable state;
FIG. 15 depicts a sectional view of a surgical instrument including a latch configured to move to an open position and engage a set of contacts to render data on a memory member unreadable;
FIG. 16 depicts a side schematic view of a circuit assembly of a surgical instrument including a memory member connected with a flex circuit to a main circuit board;
FIG. 17 depicts a side schematic view of another circuit assembly of a surgical instrument including a memory member connected with a pin connector to a main circuit board;
FIG. 18 depicts a side schematic view of a circuit assembly of a surgical instrument including a memory member with a frangible notch connected to a main circuit board;
FIG. 19A depicts a sectional view of a portion of a surgical instrument including a selectively removable shroud in an installed position, a support member, and a main circuit board in an operable state;
FIG. 19B depicts a sectional view of the portion of the surgical instrument of FIG. 19A in an uninstalled position with the shroud removed and the support member connecting the main circuit board to the shroud into an inoperable state;
FIG. 20 depicts a side view of an energy drive system of a surgical instrument with an energy coupling;
FIG. 21 depicts an enlarged side view of a portion of the energy drive system of FIG. 20;
FIG. 22 depicts a side view of an energy drive system of a surgical instrument with an energy coupling separating a waveguide from an ultrasonic transducer;
FIG. 23 depicts an enlarged sectional view of a portion of the energy drive system of FIG. 22;
FIG. 24 depicts an enlarged sectional view of a portion of another energy drive system;
Fig. 25 depicts a side view of an energy drive system including an ultrasonic transducer fitted with a cover;
FIG. 26A depicts a perspective view of a body assembly of a surgical instrument with first and second shroud portions in a connected state fitted with a strain relief feature retaining a cable and having portions thereof hidden for greater clarity; and
FIG. 26B depicts a perspective view of the body assembly of FIG. 26A with the first and second shroud portions in a disconnected state and the strain relief feature releasing the cable and having portion thereof hidden for greater clarity.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. In addition, the terms "upper," "lower," "top," "bottom," "above," and "below," are used with respect to the examples and associated figures and are not intended to unnecessarily limit the invention described herein.

### I. Example of a Surgical System

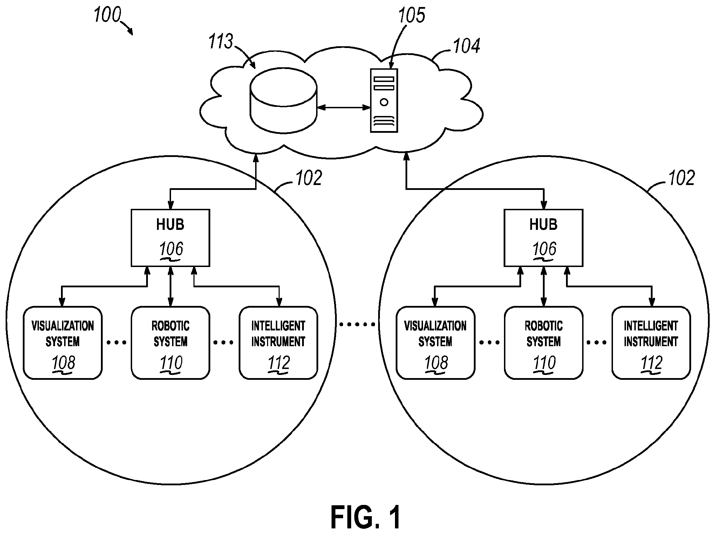

With respect to FIG. 1, a computer-implemented interactive surgical system (100) includes one or more surgical systems (102) and a cloud-based system (e.g., a cloud (104) that may include a remote server (113) coupled to a storage device (105)). Each surgical system (102) of the present example includes at least one surgical hub (106) in communication with cloud (104) that may include a remote server (113). In one example, as illustrated in FIG. 1, surgical system (102) includes a visualization system (108), a robotic system (110), and a handheld intelligent surgical instrument (112), which are configured to communicate with one another and/or hub (106). In some aspects, a surgical system (102) may include an M number of hubs (106), an N number of visualization systems (108), an O number of robotic systems (110), and a P number of handheld intelligent surgical instruments (112), where M, N, O, and P are integers greater than or equal to one.

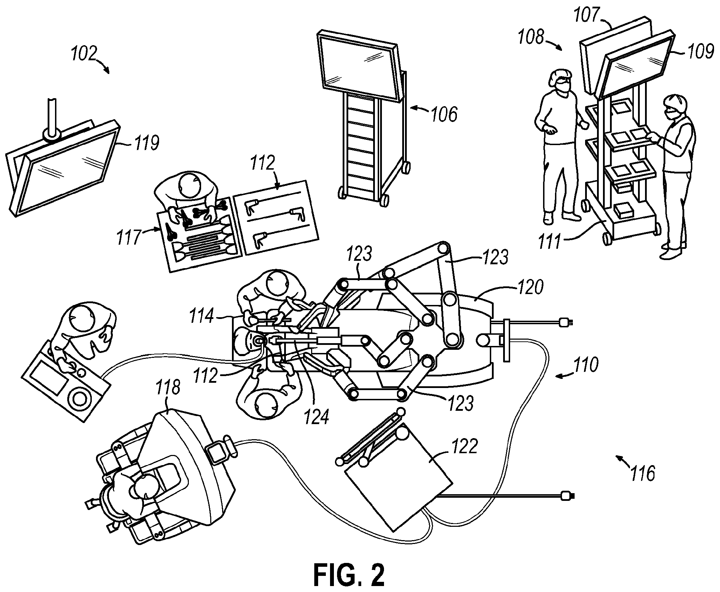

FIG. 2 depicts an example of a surgical system (102) being used to perform a surgical procedure on a patient who is lying down on an operating table (114) in a surgical operating room (116). A robotic system (110) is used in the surgical procedure as a part of surgical system (102). Robotic system (110) includes a surgeon's console (118), a patient side cart (120) (surgical robot), and a surgical robotic hub (122). Patient side cart (120) can manipulate at least one removably coupled surgical tool (117) with any one of a plurality of surgical arms (123) through a minimally invasive incision in the body of the patient while the surgeon views the surgical site through console (118). An image of the surgical site can be obtained by a medical imaging device (124), which can be manipulated by patient side cart (120) to orient imaging device (124). Robotic hub (122) can be used to process the images of the surgical site for subsequent display to the surgeon through console (118).

Other types of robotic systems can be readily adapted for use with surgical system (102). Various examples of robotic systems and surgical tools that are suitable for use with the present disclosure are described in U.S. Patent Application Publication No. 2019/0201137.

Various examples of cloud-based analytics that are performed by cloud (104, and are suitable for use with the present disclosure, are described in U.S. Patent Application Publication. No. 2019/0206569.

In various aspects, imaging device (124) includes at least one image sensor and one or more optical components. Suitable image sensors include, but are not limited to, Charge-Coupled Device (CCD) sensors and Complementary Metal-Oxide Semiconductor (CMOS) sensors. In various aspects, imaging device (124) is configured for use in a minimally invasive procedure. Examples of imaging devices suitable for use with the present disclosure include, but not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope. Some aspects of spectral and multispectral imaging are described in greater detail in U.S. Patent Application Publication. No. 2019/0200844.

Strict sterilization of the operating room and surgical equipment is required during any surgery. The strict hygiene and sterilization conditions required in a "surgical theater," i.e., an operating or treatment room, necessitate the highest possible sterility of all medical devices and equipment. Part of that sterilization process is the need to sterilize anything that comes in contact with the patient or penetrates the sterile field. It will be appreciated that the sterile field may be considered a specified area, such as within a tray or on a sterile towel, which is considered free of microorganisms, or the sterile field may be considered an area, immediately around a patient, who has been prepared for a surgical procedure. The sterile field may include the scrubbed team members, who are properly attired, and all furniture and fixtures in the area.

In addition to the introduction of any features of surgical system (100), furniture, or fixtures into the sterile field requiring sterilization, additional complications may result from removal of these features from the sterile field, particularly when such features may have contacted, or presumed to have contacted, the patient, including any tissues and/or fluids associated with the surgical procedure. Such contamination of these features from the patient often requires special consideration during or after the surgical procedure, particularly when processing these features for disposal, reuse, or remanufacturing as desired. In one example, surgical system (100) and/or healthcare professionals associated with the surgical procedure may be specifically equipped to address such processing as discussed below in greater detail.

As illustrated in FIG. 2, a primary display (119) is positioned in the sterile field to be visible to an operator at operating table (114). In addition, a visualization tower (111) is positioned outside the sterile field. Visualization tower (111) includes a first non-sterile display (107) and a second non-sterile display (109), which face away from each other. Visualization system (108), guided by hub (106), is configured to utilize displays (107, 109, 119) to coordinate information flow to operators inside and outside the sterile field. For example, hub (106) may cause visualization system (108) to display a snapshot of a surgical site, as recorded by imaging device (124), on a non-sterile display (107) or (109), while maintaining a live feed of the surgical site on the primary display (119). The snapshot on non-sterile display (107) or display (109) can permit a non-sterile operator to perform a diagnostic step relevant to the surgical procedure, for example.

In one aspect, hub (106) is also configured to route a diagnostic input or feedback entered by a non-sterile operator at visualization tower (111) to primary display (119) within the sterile field, where it can be viewed by a sterile operator at the operating table. In one example, the input can be in the form of a modification to the snapshot displayed on non-sterile display (107) or display (109), which can be routed to primary display (119) by hub (106).

Referring to FIG. 2, a surgical instrument (112) is being used in the surgical procedure as part of surgical system (102). Hub (106) is also configured to coordinate information flow to a display of the surgical instrument (112) such as in, for example, U.S. Patent Application Publication No. 2019/0200844. A diagnostic input or feedback entered by a non-sterile operator at visualization tower (111) can be routed by hub (106) to surgical instrument display (115) within the sterile field, where it can be viewed by the operator of surgical instrument (112). Example surgical instruments that are suitable for use with surgical system (102) are described in U.S. Provisional Patent Application Publication No. 2019/0200844, entitled "Interactive Surgical Platform," filed Dec. 28, 2017.

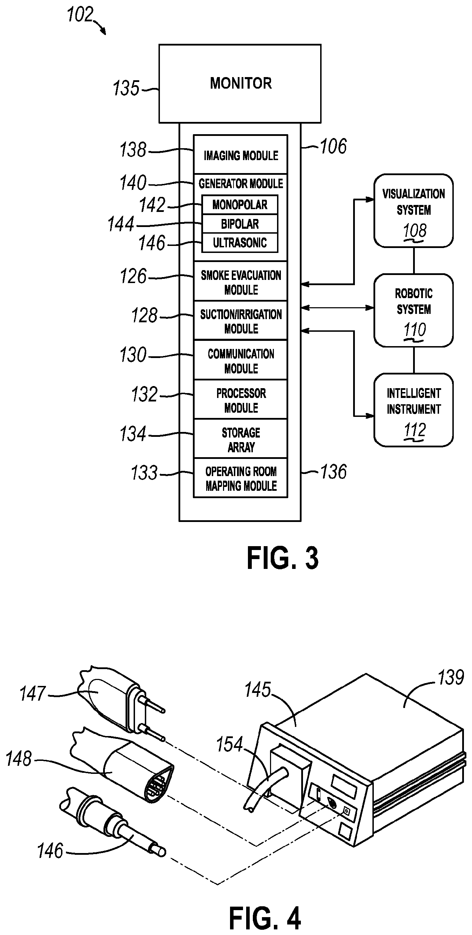

Referring now to FIG. 3, a hub (106) is depicted in communication with a visualization system (108), a robotic system (110), and a handheld intelligent surgical instrument (112). Hub (106) includes a hub display (135), an imaging module (138), a generator module (140), a communication module (130), a processor module (132), and a storage array (134). In certain aspects, as illustrated in FIG. 3, hub (106) further includes a smoke evacuation module (126) and/or a suction/irrigation module (128).

During a surgical procedure, energy application to tissue, for sealing and/or cutting, is generally associated with smoke evacuation, suction of excess fluid, and/or irrigation of the tissue. Fluid, power, and/or data lines from different sources are often entangled during the surgical procedure. Valuable time can be lost addressing this issue during a surgical procedure. Detangling the lines may necessitate disconnecting the lines from their respective modules, which may require resetting the modules. The hub modular enclosure (136) offers a unified environment for managing the power, data, and fluid lines, which reduces the frequency of entanglement between such lines.

Referring to FIGS. 3-4, aspects of the present disclosure are presented for a hub modular enclosure (136) that allows the modular integration of a generator module (140), a smoke evacuation module (126), and a suction/irrigation module (128). Hub modular enclosure (136) further facilitates interactive communication between modules (140, 126, 128). As shown in FIG. 4, generator module (140) can be a generator module with integrated monopolar, bipolar, and ultrasonic components supported in a single housing unit (139) slidably insertable into hub modular enclosure (136). As illustrated in FIG. 4, generator module (140) can be configured to connect to a monopolar device (146), a bipolar device (147), and an ultrasonic device (148). Alternatively, generator module (140) may comprise a series of monopolar, bipolar, and/or ultrasonic generator modules that interact through hub modular enclosure (136). Hub modular enclosure (136) can be configured to facilitate the insertion of multiple generators and interactive communication between the generators docked into the hub modular enclosure (136) so that the generators would act as a single generator.

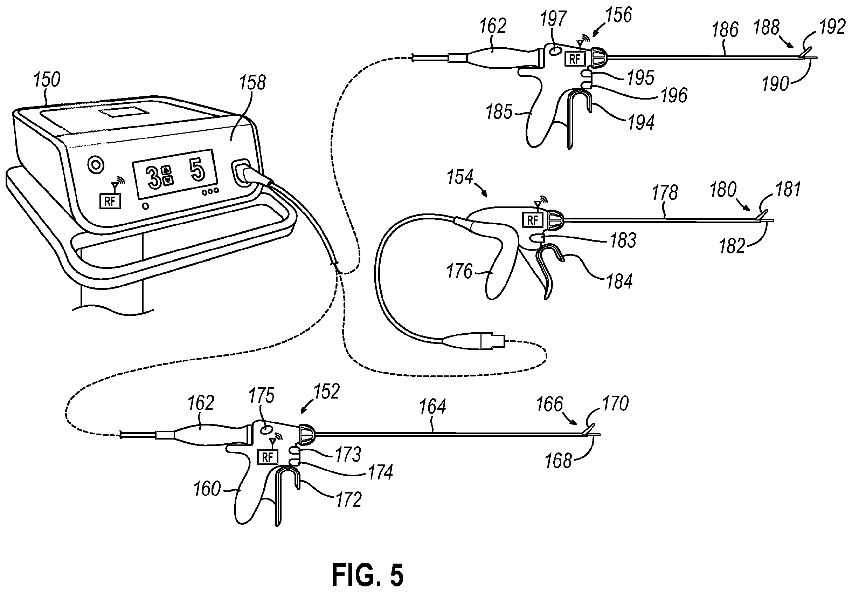

FIG. 5 illustrates one form of a generator (150) and various surgical instruments (152, 154, 156) usable therewith, where surgical instrument (152) is an ultrasonic surgical instrument (152), surgical instrument (154) is an RF electrosurgical instrument (154), and multifunction surgical instrument (156) is a combination ultrasonic/RF electrosurgical instrument (156). Generator (150) is configurable for use with a variety of surgical instruments. According to various forms, generator (150) may be configurable for use with different surgical instruments of different types including, for example, ultrasonic surgical instruments (152), RF electrosurgical instruments (154), and multifunction surgical instruments (156) that integrate RF and ultrasonic energies delivered simultaneously from generator (150). Although generator (150) of the present example in FIG. 5 is shown separate from surgical instruments (152, 154, 156), generator (150) may alternatively be formed integrally with any of surgical instruments (152, 154, 156) to form a unitary surgical system. Generator (150) comprises an input device (158) located on a front panel of generator (150) console. Input device (158) may comprise any suitable device that generates signals suitable for programming the operation of generator (150). Generator (150) may be configured for wired or wireless communication.

Generator (150) of the present example is configured to drive multiple surgical instruments (152, 154, 156). One example of such surgical instrument is ultrasonic surgical instrument (152) and comprises a handpiece (160), an ultrasonic transducer 162, a shaft assembly (164), and an end effector (166). End effector (166) includes an ultrasonic blade (168) acoustically coupled to ultrasonic transducer (162) and a clamp arm (170). Handpiece (160) has a trigger (172) to operate clamp arm (170) and a combination of toggle buttons (173, 174, 175) to energize and drive ultrasonic blade (168) or other function. Toggle buttons (173, 174, 175) can be configured to energize ultrasonic transducer (162) with generator (150).

Generator (150) also is configured to drive another example of surgical instrument (154). RF electrosurgical instrument (154) includes a handpiece (176), a shaft assembly (178), and an end effector (180). End effector (180) includes electrodes in clamp arms (181, 182) and return through an electrical conductor portion of shaft assembly (178). Electrodes are coupled to and energized by a bipolar energy source within generator (150). Handpiece (176) includes a trigger (183) to operate clamp arms (181, 182) and an energy button (184) to actuate an energy switch to energize electrodes in end effector (180).

Generator (150) also is configured to drive multifunction surgical instrument (156). Multifunction surgical instrument (156) includes a handpiece (185), a shaft assembly (186), and an end effector (188). End effector (188) has an ultrasonic blade (190) and a clamp arm (192). Ultrasonic blade (190) is acoustically coupled to ultrasonic transducer (162). Handpiece (185) has a trigger (194) to operate clamp arm (192) and a combination of toggle buttons (195, 196, 197) to energize and drive ultrasonic blade (190) or other function. Toggle buttons (195, 196, 197) can be configured to energize ultrasonic transducer (162) with generator (150) and energize ultrasonic blade (190) with a bipolar energy source also contained within generator (150). It will be appreciated that handpieces (160, 176, 185) may be replaced with a robotically controlled instrument for incorporating one or more aspects of surgical instruments (152, 154, 156). Accordingly, the term "handpiece" should not be limited to this context and to handheld use.

As used throughout this description, the term "wireless" and its derivatives may be used to describe circuits, devices, systems, methods, techniques, communications channels, etc., that may communicate data through the use of modulated electromagnetic radiation through a non-solid medium. The term does not imply that the associated devices do not contain any wires, although in some aspects they might not. The communication module may implement any of a number of wireless or wired communication standards or protocols, including but not limited to Wi-Fi (IEEE 802.11 family), WMAX (IEEE 802.16 family), IEEE 802.20, long term evolution (LTE), Ev-DO, HSPA+, HSDPA+, HSUPA+, EDGE, GSM, GPRS, CDMA, TDMA, DECT, Bluetooth, Ethernet derivatives thereof, as well as any other wireless and wired protocols that are designated as 3G, 4G, 5G, and beyond. The computing module may include a plurality of communication modules. For instance, a first communication module may be dedicated to shorter range wireless communications such as Wi-Fi and Bluetooth and a second communication module may be dedicated to longer range wireless communications such as GPS, EDGE, GPRS, CDMA, WiMAX, LTE, Ev-DO, and others.

As used herein a processor or processing unit is an electronic circuit which performs operations on some external data source, usually memory or some other data stream. The term is used herein to refer to the central processor (central processing unit) in a system or computer systems (especially systems on a chip (SoCs)) that combine a number of specialized "processors."

As used herein, a system on a chip or system on chip (SoC or SOC) is an integrated circuit (also known as an "IC" or "chip") that integrates all components of a computer or other electronic systems. It may contain digital, analog, mixed-signal, and often radiofrequency functions, all on a single substrate. A SoC integrates a microcontroller (or microprocessor) with advanced peripherals like graphics processing unit (GPU), Wi-Fi module, or coprocessor. A SoC may or may not contain built-in memory.

As used herein, a microcontroller or controller is a system that integrates a microprocessor with peripheral circuits and memory. A microcontroller (or MCU for microcontroller unit) may be implemented as a small computer on a single integrated circuit. It may be similar to a SoC; an SoC may include a microcontroller as one of its components. A microcontroller may contain one or more core processing units (CPUs) along with memory and programmable input/output peripherals. Program memory in the form of Ferroelectric RAM, NOR flash or OTP ROM is also often included on chip, as well as a small amount of RAM. Microcontrollers may be employed for embedded applications, in contrast to the microprocessors used in personal computers or other general-purpose applications consisting of various discrete chips.

As used herein, the term controller or microcontroller may be a stand-alone IC or chip device that interfaces with a peripheral device. This may be a link between two parts of a computer or a controller on an external device that manages the operation of (and connection with) that device. Modular devices include the modules (as described in connection with FIG. 3, for example) that are receivable within a surgical hub and the surgical devices or instruments that can be connected to the various modules in order to connect or pair with the corresponding surgical hub. The modular devices include, for example, intelligent surgical instruments, medical imaging devices, suction/irrigation devices, smoke evacuators, energy generators, ventilators, insufflators, and displays. The modular devices described herein can be controlled by control algorithms. The control algorithms can be executed on the modular device itself, on the surgical hub to which the particular modular device is paired, or on both the modular device and the surgical hub (e.g., via a distributed computing architecture). In some exemplifications, the modular devices' control algorithms control the devices based on data sensed by the modular device itself (i.e., by sensors in, on, or connected to the modular device). This data can be related to the patient being operated on (e.g., tissue properties or insufflation pressure) or the modular device itself (e.g., the rate at which a knife is being advanced, motor current, or energy levels). For example, a control algorithm for a surgical stapling and cutting instrument can control the rate at which the instrument's motor drives its knife through tissue according to resistance encountered by the knife as it advances.

### II. Exemplary Surgical Instrument Incorporating Selectively Separable Shroud

In some instances, it may be desirable to provide a surgical instrument (1000) similar to any one or more of surgical instruments (112, 152, 154, 156) that includes components capable of delivering ultrasonic energy, RF energy, or both ultrasonic and RF energy that easily open to provide access to the internal components for separation into separate waste streams with minimal tools, such as no additional tools. Surgery customarily takes place within the sterile field, as described above. The sterile field, being free of microorganisms, enables the surgical team to decrease the chance of infection by ensuring that only sterilized equipment and tools are used within the sterile field. Surgical instruments are sterilized and packaged within sterile containers that are passed into the sterile field. Health care professionals may be required to disassemble the surgical instruments within the sterile field after a surgical procedure by hand or with tools provided within the sterile containers. For example, a torque wrench provided for assembling a surgical instrument, may have additional features to disassemble the surgical instrument. The surgical instruments include additional features that facilitate disassembly and removal of internal components. These additional features aid in selectively breaking the internal components so the components may be placed into separate waste streams. These separate waste streams are predetermined based on the material of the component or the use of the component. For example, the waste streams may include recycling, disposal, or refurbishing. Components placed in the disposal waste stream would be disposed of in a land fill. Components placed in the recycling waste stream may be further separated, shredded, and melted down into a base component. Components placed in the refurbishing waste stream would be cleaned, tested, repaired, and refitted within another surgical instrument. For example, the plastic and metal components of a shroud may be separated into one waste stream for disposal, heavy metals from an integrated circuit may be separated into a second waste stream for recycling, and ultrasonic transducers may be separated into a third waste stream for refurbishing.

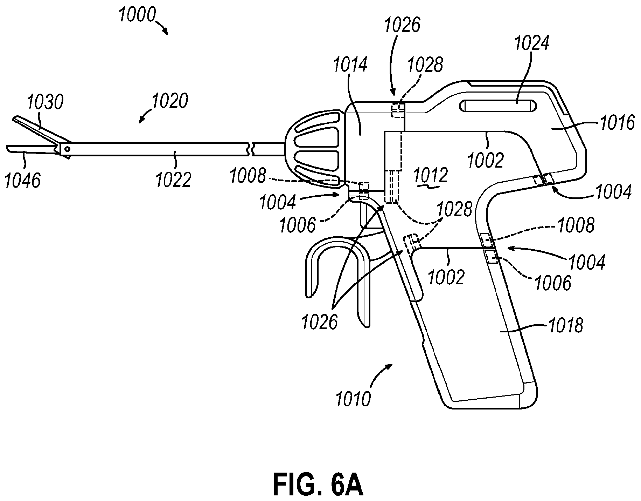

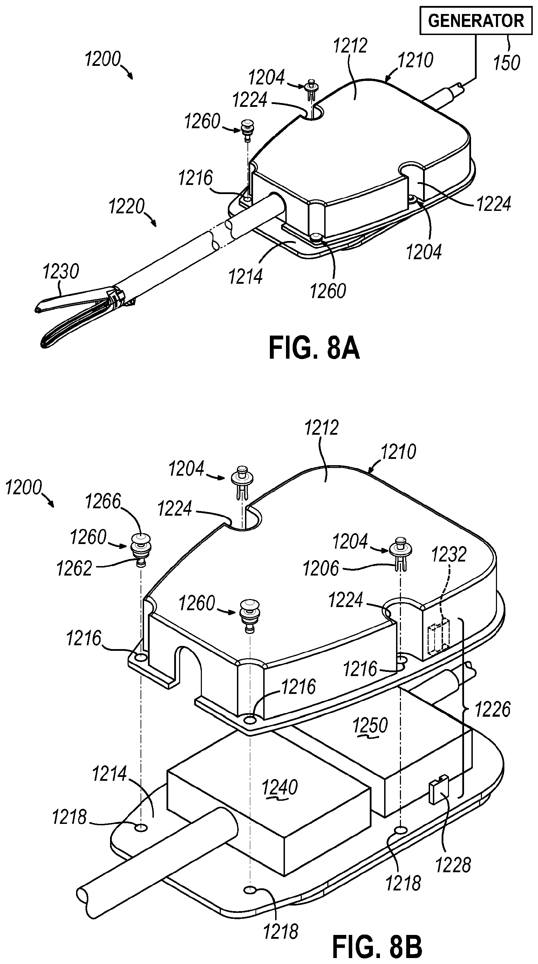

FIGS. 6A-6B illustrate one example of a surgical instrument (1000) similar to surgical instruments (112, 152, 154, 156) configured to treat tissue. Surgical instrument (1000) may be configured to deliver ultrasonic energy, Radio Frequency ("RF") energy, or both. Additionally, surgical instrument may be configured to be hand-held or fitted with a corresponding portion of a robotic arm (*see* FIG. 8A). Surgical instrument (1000), like surgical instruments (112, 152, 154, 156), includes a body assembly (1010), a shaft assembly (1020) and an end effector (1030). Shaft (1022) of shaft assembly (1020) extends distally from body assembly (1010) to end effector (1030). Surgical instrument (1000) differs from surgical instruments (112, 152, 154, 156) in that surgical instrument (1000) includes body assembly (1010) configured to be easily disassembled to expose at least one of a plurality of internal components for removal and disposal into separate waste streams.

Surgical instrument (1000) of the present example is configured to deliver ultrasonic energy similar to surgical instrument (152). Body assembly (1010) surrounds a portion of an energy drive system (1040) and a portion of a circuit assembly (1050). Energy drive system (1040), in the present version, includes an ultrasonic transducer (1042), a waveguide (1044), and an ultrasonic blade (1046). Energy drive system (1040) may further include a battery (1048), or a generator (150) (*see* FIG. 5) configured to supply energy. Ultrasonic transducer (1042) is proximally positioned within body assembly (1010) and extends distally to waveguide (1044). Waveguide (1044) extends distally through shaft assembly (1020) to ultrasonic blade (1046). Circuit assembly (1050) includes a main circuit board (1052), a memory member (1054), and a controller (1056). Circuit assembly (1050) may be in electrical communication with a power supply such as battery (1048) or generator (150) (*see* FIG. 5) and is operatively connected to energy drive system (1040).

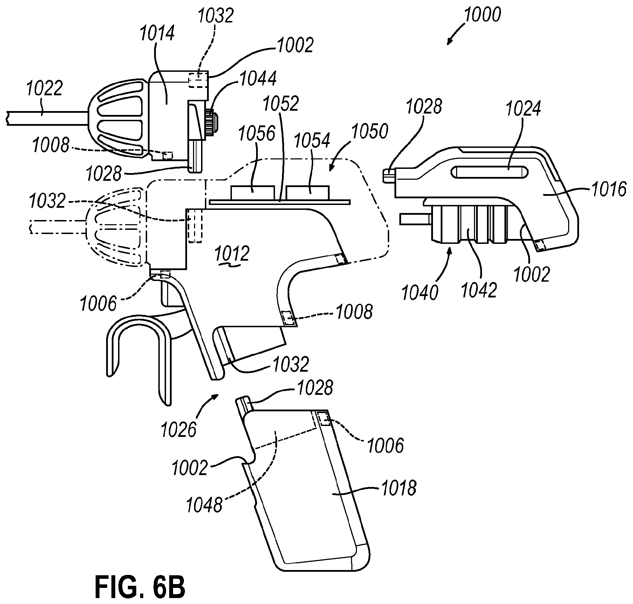

Body assembly (1010) includes a plurality of selectively removeable shroud portions (1012, 1014, 1016, 1018). Shroud portions (1012, 1014, 1016, 1018) are configured to provide support for energy drive system (1040), shaft assembly (1020), and circuit assembly (1050). Shroud portions (1012, 1014, 1016, 1018) additionally inhibit access to a portion of energy drive system (1040) and a portion of circuit assembly (1050). As illustrated, shroud portions (1012, 1014, 1016, 1018) include a first shroud portion (1012), a second shroud portion (1014), a third shroud portion (1016), and a fourth shroud portion (1018), but may include any number of shroud portions (1012, 1014, 1016, 1018) that inhibit access to circuit assembly (1050) and energy drive system (1040). This configuration of shroud portions (1012, 1014, 1016, 1018) is merely one example and not intended to unnecessarily limit the invention. Each shroud portion (1012, 1014, 1016, 1018) is removably affixed to another shroud portion (1012, 1014, 1016, 1018) at a shroud edge (1002). Shroud portions (1012, 1014, 1016, 1018) are mated together at shroud edges (1002) with shroud couplings (1004). Shroud couplings (1004) connect two adjacent shroud edges (1002) during normal operation of surgical instrument (1000) in a connected state. Magnetic fastener in the form of a shroud coupling (1004) includes a first magnetic member (1006) and a second magnetic member (1008). One of first magnetic member (1006) or second magnetic member (1008) includes a rare earth magnet or an electromagnet. Other of first or second magnetic member (1006, 1008) includes rare earth magnet, electromagnet, or a ferromagnetic metal. Ferromagnetic metals include but are not limited to iron, cobalt, or nickel. First magnetic member (1006) is attracted to second magnetic member (1008) by a magnetic field (MF). Magnetic field (MF) includes sufficient force to retain adjacent shroud edges (1002) of shroud portions (1012, 1014, 1016, 1018) in a connected state during operation, but allows for a user to transition shroud portions (1012, 1014, 1016, 1018) to a disconnected state (*see* FIG. 6B) after operation. Users may remove a shroud portion (1012, 1014, 1016, 1018) to provide access to portions of energy drive system (1040) and portions of circuit assembly (1050) in a disconnected state (*see* FIG. 6B). Once accessed, portions of energy drive system (1040) and portions of circuit assembly (1050) may be disposed of in separate waste streams. It should be noted that shroud portions (1012, 1014, 1016, 1018) may include gripping features (1024) positioned on exterior of shroud portions (1012, 1014, 1016, 1018) to facilitate opening of shroud portions (1012, 1014, 1016, 1018).

Shroud portions (1012, 1014, 1016, 1018) further include a plurality of alignment features (1026) configured to align each shroud portion (1012, 1014, 1016, 1018) with an adjacent shroud portion (1012, 1014, 1016, 1018). Alignment features (1026) facilitate translating shroud portions (1012, 1014, 1016, 1018) from connected state to the disconnected state (*see* FIG. 6B) and prevent binding of shroud portions (1012, 1014, 1016, 1018) when removed from one another. Additionally, alignment features (1026) facilitate alignment of first magnetic member (1006) and second magnetic member (1008) when assembling surgical instrument (1000). Alignment features (1026) in one example include a key (1028) positioned on one of first shroud portion (1012) or second shroud portion (1014) and a keyway (1032) positioned on other of first shroud portion (1012) or second shroud portion (1014). Key (1028) is sized to slide within keyway (1032). Key (1028) and keyway (1032) include complementary shapes such as circular, rectangular, square, or triangular. Shroud edges (1002) may overlap to mate key (1028) with keyway (1032) or at least one of key (1028) or keyway (1032) may extend past one of shroud edges (1002) to mate with other of key (1028) or keyway (1032).

FIG. 6B shows surgical instrument (1000) after being transitioned to the disconnected state. In the disconnected state, shrouds (1012, 1014, 1016, 1018) are separated from one another. For example, third shroud (1016) is manually moved horizontally away from first shroud (1012). Second shroud (1014) is manually moved vertically away from first shroud (1012). Fourth shroud (1018) is manually moved slantwise away from first shroud (1012) separating shroud coupling (1004). Alignment features (1026) facilitate the movement of transducer shroud in a horizontal path, second shroud (1014) in a vertical path, and fourth shroud (1018) in a slantwise path. Removal of shrouds (1012, 1014, 1016, 1018) facilitates access to at least a portion of energy drive system (1040) and at least a portion of circuit assembly (1050).

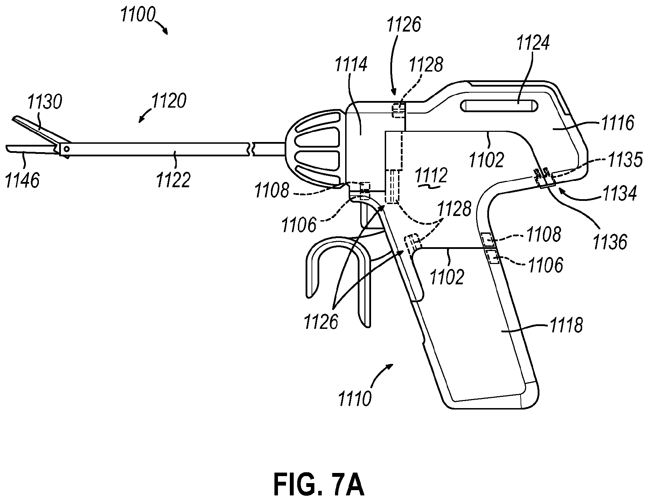

FIG. 7A illustrates one form of a surgical instrument (1100) that is similar to surgical instrument (1000) except as otherwise described herein. Surgical instrument (1100) is shown in a connected state. Surgical instrument (1100), like surgical instrument (1000), includes a body assembly (1110), a shaft assembly (1120) and an end effector (1130). Body assembly (1110) is proximally located relative to shaft assembly (1120). Shaft assembly (1120) includes a shaft (1122) distally extending from body assembly (1110) to an end effector (1130). End effector (1130) includes an ultrasonic blade (1146). Body assembly (1110) includes a plurality of shroud portions (1112, 1114, 1116, 1118) configured to inhibit access to a portion of an energy drive system (1140) and a portion of a circuit assembly (1150). Shroud portions (1112, 1114, 1116, 1118) are connected by a magnetic fastener in the form of a magnetic lock assembly (1134) at complementary shroud edges (1102) of shroud portions (1112, 1114, 1116, 1118). Magnetic lock assembly (1134) includes a first magnetic member (1106), a second magnetic member (1108), a magnetic lock (1135), and a lock key (1136). Magnetic lock assembly (1134) differs from shroud coupling (1004) of surgical instrument (1000) in that magnetic lock assembly (1134) is configured to prevent inadvertently moving shroud portion (1112, 1114, 1116, 1118) away from another shroud portion (1112, 1114, 1116, 1118) without further action by the user. First and second magnetic members (1106, 1108) may include a rare earth magnet, an electromagnet, or a ferromagnetic metal. Magnetic lock (1135) may also be configured as a solenoid (not shown). Solenoid may include an electromagnet and a ferromagnetic metal rod. Solenoid may be positioned on shroud portion (1112, 1114, 1116, 1118) with a strike plate (not shown) having a bore or an aperture on another shroud portion (1112, 1114, 1116, 1118). Ferromagnetic rod is positioned within the strike plate and maintains shroud portions (1112, 1114, 1116, 1118) in a connected state. Solenoid may be configured to move ferromagnetic rod transversely, longitudinally, or slantwise relative to shroud edge (1102) and into strike plate. In some versions, circuit assembly (1150) may be configured to be in electrical communication with magnetic lock (1135). Each shroud edge (1102) of shroud portions (1112, 1114, 1116, 1118) may be fitted with magnetic lock (1135) controlled by circuit assembly (1150). Circuit assembly (1150) in such versions, is able to transition all magnetic locks (1135) from a locked state to an unlocked state and vise-versa.

Magnetic lock assembly (1134) further includes lock key (1136) that in the present example is separable from magnetic lock (1134) and the magnetic members (1106, 1108) are separate and apart from the magnetic lock (1134). In the present versions, lock key (1136) removes electricity from an electromagnet thereby removing magnetic field, which stops the magnetic attraction between first and second magnetic members (1106, 1108) when lock key (1136) is removed from the magnetic lock assembly (1134). In the alternative, lock key (1136) may be inserted into the magnetic lock assembly (1134) to transition magnetic locks (1135) from a locked state to an unlocked state. Lock key (1136) may remove the electricity by shorting an electrical circuit (not shown), breaking an electrical circuit (not shown), or by energizing a switch (not shown).

In some versions, lock key (1136) transitions magnetic lock (1135) from the locked state to the unlocked stated by physically moving one of first or second magnetic members (1106, 1108) away from other of first or second magnetic members (1106, 1108), thereby reducing the magnetic attraction between first and second magnetic members (1106, 1108). In such versions, magnetic lock (1135), first magnetic member (1106), and second magnetic member (1108) are proximate to one another. This reduced magnetic attraction or lack of magnetic attraction allows a user to manually separate shroud portions (1112, 1114, 1116, 1118).

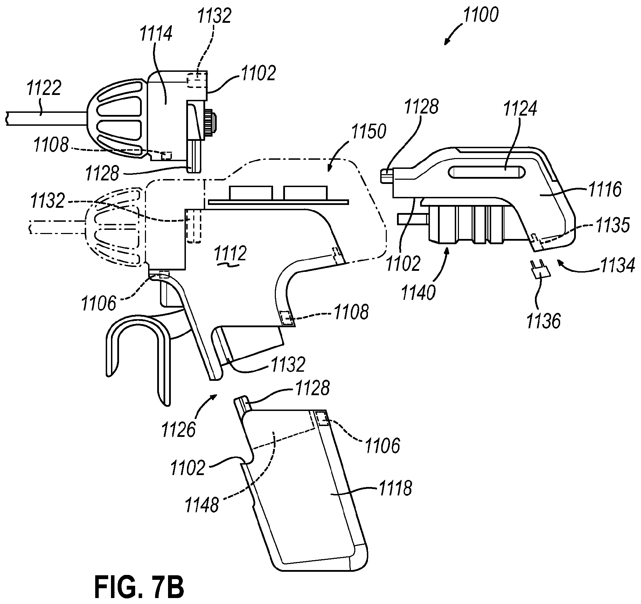

FIG. 7B shows surgical instrument (1100) in the disconnected state after magnetic lock assembly (1134) has been transitioned from the locked state to the unlocked state. Body assembly further includes a gripping feature (1124) configured to facilitate grabbing shroud portions (1112, 1114, 1116, 1118) to separate shroud portions (1112, 1114, 1116, 1118) from one another and an alignment feature (1126) such as a key (1128) and keyway (1132) configured to aid in aligning shroud portions (1112, 1114, 1116, 1118) when removed from one another. In the present version, alignment feature is separate and apart from magnetic lock assembly (1134) and/or magnetic members (1108, 1106). In some versions, alignment feature (1126) is incorporated into magnetic lock assembly (1134) and/or magnetic members (1108, 1106).

FIGS. 8A-10 illustrate one form of a surgical instrument (1200) similar to surgical instrument (1000) configured to deliver electrical energy to treat tissue. Surgical instrument (1200) as illustrated is configured to be fitted to a corresponding portion of a robotic arm but may also be configured as a hand-held surgical instrument as illustrated in FIGS. 6A-7B. Surgical instrument (1200) is shown in electrical communication with a generator (150) (*see* Fig. 5). Generator (150) is configured to supply energy to surgical instrument (1200). Surgical instrument (1200), like surgical instrument (1000), includes a body assembly (1210), a shaft assembly (1220), and an end effector (1230). Shaft assembly (1220) extends distally from body assembly (1210) to end effector (1230).

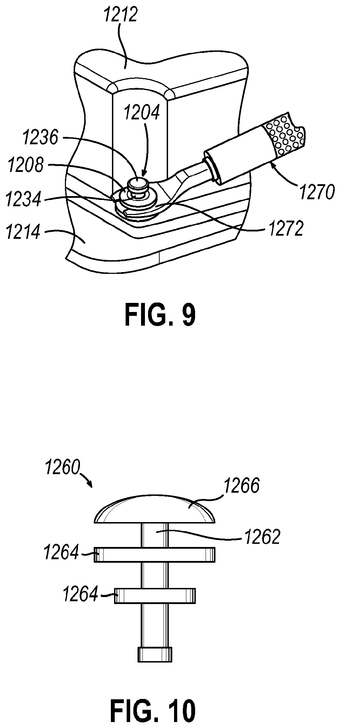

Body assembly (1210) is configured to be disassembled to expose a portion of an energy drive system (1240) and a portion of a circuit assembly (1250). Body assembly (1210) includes a plurality of shroud portions (1212, 1214) having an upper shroud portion (1212) and a lower shroud portion (1214) configured to be coupled together with a shroud coupling in the form of push-pins (1204, 1260). Push-pins (1204, 1260) may be constructed of nylon or some other material known in the art to have resilient properties. More particularly, push-pin (1204) is a two-piece push-pin (1204), whereas push-pin (1260) is a single piece push-pin (1260). Two-piece push-pin (1204) of the present example includes a shank (1206), a shank head (1208) (*see* FIG. 9), a pin (1234) (*see* FIG. 9), and a pin head (1236) (*see* FIG. 9). One-piece push-pin (1260) of the present example includes a shank (1262) and a plurality of resilient ribs (1264) (*see* FIG. 10) positioned along shank (1262) and a shank head (1266) positioned at a proximal end of shank (1262). Upper shroud portion (1212) defines an upper bore (1216), and lower shroud portion (1214) defines a lower bore (1218). FIG. 8A shows upper bore (1216) aligned with lower bore (1218) and fitted with a push-pins (1204, 1260) in the connected state. Push-pin (1204) forces upper shroud portion (1212) against lower shroud portion (1214) to retain upper shroud portion (1212) against lower shroud portion (1214) in the connected state. It should be noted that surgical instrument (1200) may include all of one type of push-pin (1204, 1206) or include more than one type of push-pin (1204, 1206). In the present version, surgical instrument (1200) includes both two-piece push-pins (1204) and one-piece push-pins (1206).

Two-piece push-pin (1204) is installed by first inserting shank (1206) through upper and lower bores (1216, 1218) so that shank head (1208) rests on a surface adjacent to upper bore (1216). Before inserting, shank (1206) remains in an unexpanded state sized to be fitted within upper and lower bore (1216, 1218). Pin (1234) is pressed within a bore of shank (1206) until pin head (1236) is seated upon a top of shank head (1208). In the installed position, a distal portion of shank (1206) is expanded by pin (1234) to an expanded state and has a diameter that is larger than upper and lower bores (1216, 1218). The expanded shank (1206) axially pulls upper shroud portion (1212) towards lower shroud portion (12140). Installing pin (1234) distally into shank (1206) results in distal portion of shank (1206) having a larger outer diameter than distal portion of shank (1206) before pin (1234) was installed into shank (1206).

One-piece push-pin (1260) is installed by pressing on top of shank head (1266) while directing a distal end of shank (1262) into upper and lower bores (1216, 1218). One-piece push-pin (1260) is installed with shank head (1266) being positioned on a first side of upper bore (1216) and a proximal-most rib (1264) being positioned on a distal side of lower bore (1218) such that push-pin (1260) resists removal of upper shroud portion (1212) from lower shroud portion (1214).

Other versions of push-pins (not shown) may be configured for manual operation without additional tools. These other versions operate similar to a blind rivet but having an actuator (not shown), a spring (not shown), a pin (not shown) and a shank (not shown). Actuator is moved by a user translating pin within shank (not shown). An outside diameter of shank is reduced so that push-pin may be retracted through upper and lower bores (1216, 1218) and upper and lower shroud portions (1216, 1218) are transitioned from a connected state to a disconnected state.

FIG. 8B shows surgical instrument (1200) in the disconnected state with push-pins (1204, 1260) removed from upper and lower bores (1216, 1218). In the disconnected state, body assembly (1210) provides access to a portion of energy drive system (1240) and a portion of circuit assembly (1250) for removal and disposal in separate waste streams. Upper and lower shroud portions (1212, 1214) may include gripping features (1224) that aid in removal of upper shroud portion (1212) from lower shroud portion (1214) and alignment features (1226) that provide alignment of upper and lower bores (1216, 1218). Alignment features (1226) such as a key (1228) positioned on one of the upper or lower shroud portions (1212, 1214) and a keyway (1232) positioned on the other of the upper or lower shroud portions (1212, 1214).

FIG. 9 shows push-pin (1204) being removed from upper and lower shroud portions (1212, 1214) by a torque wrench (1270) including a forked member (1272). Push-pin (1204) may be constructed of nylon or some other material known in the art to have resilient properties.

FIG. 10 shows one-piece push-pin (1260) including shank (1262) and the plurality of resilient ribs (1264) positioned along shank (1262) and shank head (1266) positioned at a proximal end of shank (1262). Push-pin (1260) is also constructed of nylon or some other material known in the art to have resilient properties. Push-pin (1260) may also be removed with torque applied to push-pin (1260) by prying upon shank head (1266) with forked member (1272).

### III. Exemplary Surgical Instrument Incorporating Selectively Separable Circuit Assemblies

In some instances, it may be desirable to provide a surgical instrument that includes components capable of delivering ultrasonic energy, RF energy, or both ultrasonic and RF energy that is easily opened so that the internal components may be separated into separate waste streams with minimal tools, such as no tools, within the sterile field. These surgical instruments are configured to stay intact during normal use but facilitate disassembly of internal components and/or selectively break the internal components. One such internal component is a circuit assembly that is configured to be separated by a hand or hands of the user into separate portions having different properties. The separate portions of the circuit assembly are placed in predetermined separate waste streams. These waste streams include but are not limited to recycling, disposal, or refurbishing.

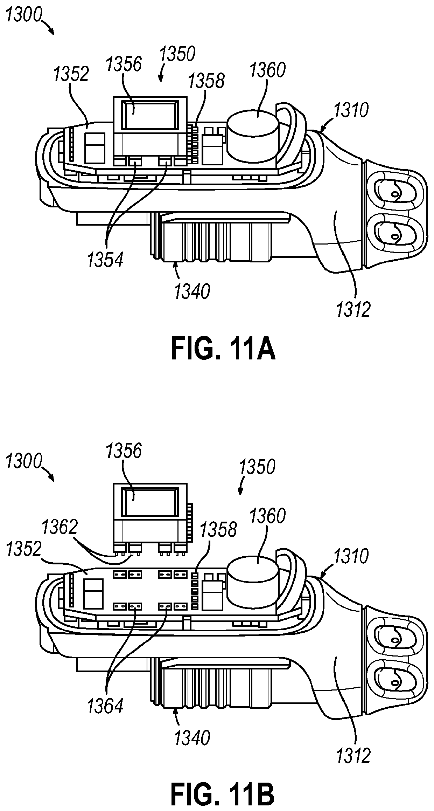

FIGS. 11A-11B show a portion of surgical instrument (1300) similar to surgical instrument (1000) except as otherwise described herein. Surgical instrument (1300) includes a shaft assembly (not shown) that extends distally from a body assembly (1310) to an end effector (not shown). Body assembly (1310) includes a plurality of shroud portions (1312) similar to surgical instrument (1000). Top shroud portion (not shown) has been removed from lower shroud portion (1312) to expose a portion of circuit assembly (1350). Circuit assembly (1350) includes a main circuit board (1352) and a plurality of sub-boards (1354). Lower shroud portion (1312) provides support for circuit assembly (1350) and energy drive assembly (1340). Main circuit board (1352) includes an integrated circuit (1358) configured to provide electrical communication between a memory member (1356), a controller (1360), inputs (not shown), and outputs (not shown). In some versions, sub-boards (1354) include memory member (1356) and controller (1360). In the present version, main circuit board (1352) includes an integrated controller (1360) and sub-boards (1354) include memory member (1356). FIG. 11A shows sub-boards (1354) plugged into main circuit board (1352) in an installed position in communication with controller (1360) via integrated circuit (1358).

FIG. 11B shows sub-boards (1354) having memory member (1356) shown in an uninstalled position. Memory member (1356) in the uninstalled position is unplugged from main circuit board (1352). Memory member (1356) may include electrically erasable programmable read-only memory ("EEPROM"), erasable programable read-only memory ("EPROM"), programable read-only memory ("PROM"), read only memory ("ROM"), random access memory ("RAM"), or other suitable forms of memory known in the art for use with circuit assemblies. Sub-boards (1354) include a plurality of prongs (1362) configured to removably couple with respective receptacles (1364) defined by main circuit board (1352). Placement of prongs (1362) and receptacles (1364) may be reversed with prongs (1362) being located on main circuit board (1352) and receptacle (1364) being located on sub-board (1354). Prongs (1362) provide electrical communication between sub-boards (1354) and main circuit board (1352). Sub-boards (1354) may be removed from main circuit board (1352) for disposal in a separate waste stream than main circuit board (1352). For example, sub-boards (1354) may be refurbished and reused, and main circuit board (1352) may be recycled, although such distribution is merely one example and not intended to unnecessarily limit the invention.

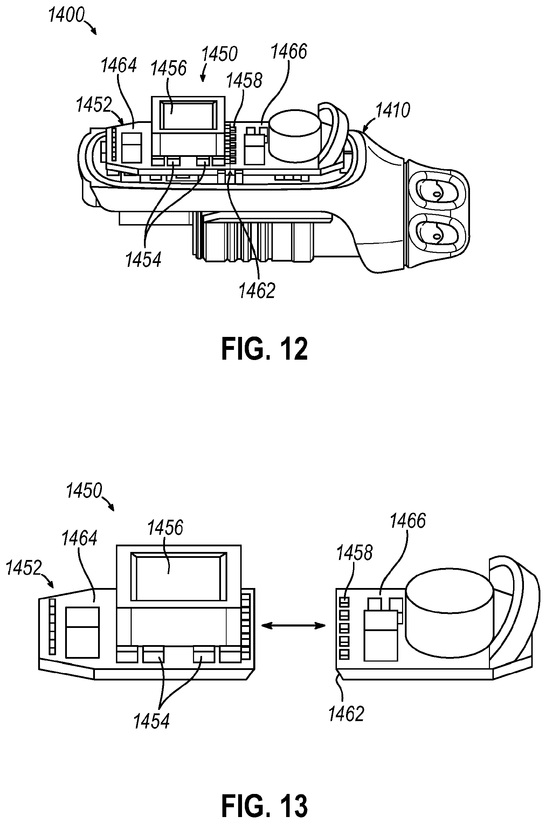

FIGS. 12-13 show a portion of surgical instrument (1400) similar to surgical instrument (1300) except as otherwise described herein. Surgical instrument (1400) like surgical instrument (1300) includes a circuit assembly (1450) configured to be broken into separate portions for disposal in separate waste streams. FIG. 12 shows a portion of body assembly (1410) with a top shroud portion (not shown) removed to expose circuit assembly (1450). Circuit assembly (1450) includes a main circuit board (1452) and sub-boards (1454). Main circuit board (1452) and sub-boards (1454) are shown in an intact state. Surgical instrument (1400) differs from surgical instrument (1300) in that sub-boards (1454) are fixedly coupled to main circuit board (1452) and main circuit board (1452) includes frangible separators (1462) configured to connect a first circuit portion (1464) to a second circuit portion (1466) in an operable state. Frangible separators (1462) may include perforations, consecutive holes, weakened portions, or any other separation feature know in the art that would facilitate breaking a circuit board along a predetermined path. In the operable state, frangible separators (1462) allow for electrical communication along circuit assembly (1450), such as electrical communication across frangible separators (1462).

FIG. 13 shows circuit assembly (1450) in a separated state with first circuit portion (1464) separated from second circuit portion (1466). First circuit portion (1464) may be separated from second circuit portion (1466) by breaking frangible separator (1462), such as with a user's hand or hands. First circuit portion (1464) may include components that require first circuit portion (1664) to be placed in a separate waste stream than second circuit portion (1466). For example, sub-board (1454) may have memory member (1456) unsuitable for reconditioning or recycling that is placed in the disposal waste stream and second circuit portion (1466) may include integrated circuit (1458) including heavy metals that require reconditioning or recycling, although such arrangement is merely one example and not intended to unnecessarily limit the invention.

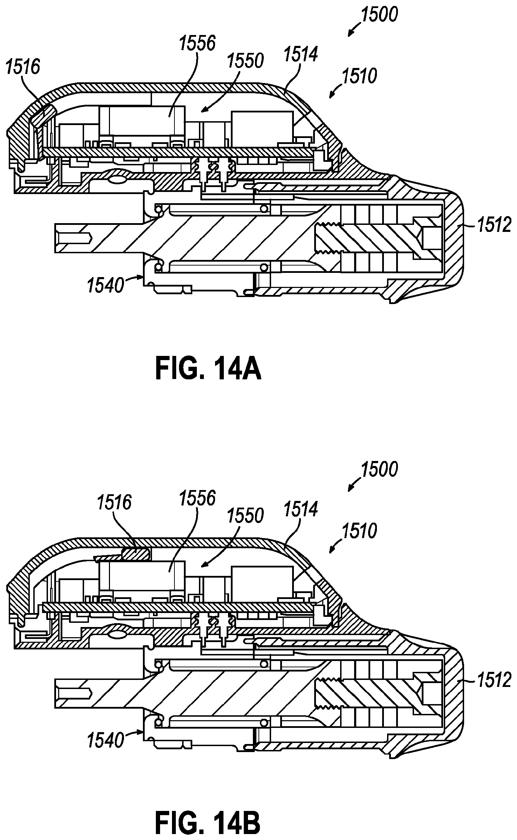

FIGS. 14A-14B show a portion of surgical instrument (1500) similar to surgical instrument (1400) except as otherwise described herein. A body assembly (1510) of the present example includes a first shroud portion (1512), a second shroud portion (1514), and a latch (1516). First shroud portion (1512) provides support for a portion of energy drive system (1540) and a portion of a circuit assembly (1550). Second shroud portion (1514) inhibits access to circuit assembly (1550). Second shroud portion (1514) is removably affixed to first shroud portion (1512) with latch (1516) in a secured position. FIG. 14A shows circuit assembly (1550) having a memory member (1556) in an operable state with latch (1516) in a closed position with second shroud portion (1514) connected to first shroud portion (1512). Memory member (1556) as mentioned above may include RAM, ROM, PROM, EPROM, EEPROM, or any other suitable forms of memory known in the art.

FIG. 14B shows the circuit assembly (1550) in an inoperable state after latch (1516) was transitioned to an open position. Latch (1516) includes a rare earth magnet that uses a magnetic field to causes a disruption of electrical signals or magnetic pulse that damages or scrambles memory within memory member (1556). Latch (1516) transitions from a secured position to an unsecured position. Before fully opening second shroud portion (1514), latch (1516) passes in close proximity to memory member (1556) thereby rendering data contained in memory member (1556) unreadable. By way of example, latch (1516) may have a hall effect sensor with an integrated magnet that allows circuit assembly (1550) initiate a sequence rendering the data unreadable, such as a reset, rewrite, or scramble, which erases or destroys memory. In other versions, the latch (1516) includes a magnet that passed a magnetic field close to the memory member (1556) that erases or destroys the memory.

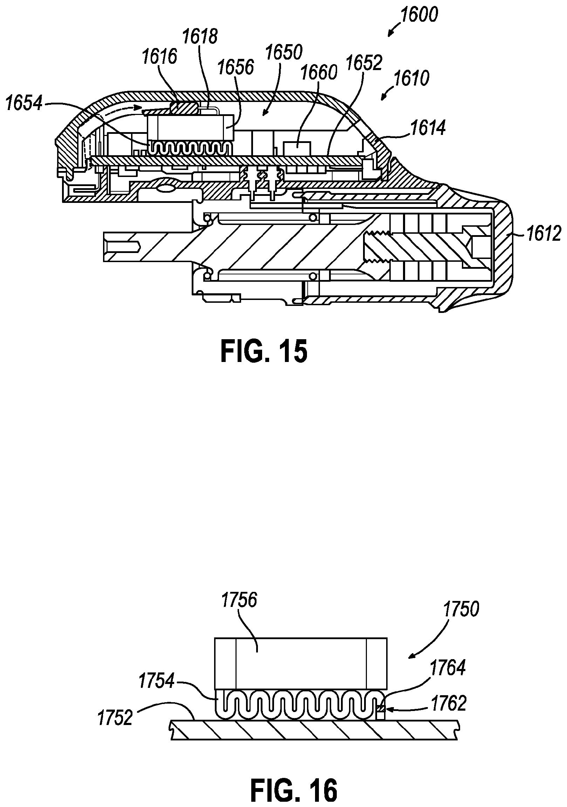

FIG. 15 shows a portion of surgical instrument (1600) similar to surgical instrument (1500) except as otherwise described herein. Like surgical instrument (1500), surgical instrument (1600) includes a body assembly (1610) having a first shroud portion (1612) connected to a second shroud portion (1614) by a latch (1616). Body assembly (1610) houses, supports, and inhibits access to circuit assembly (1650). Circuit assembly (1650) includes a memory member (1656), a main circuit board (1652), and a flexible circuit (1654). Memory member (1656) as mentioned above may include a memory such as RAM, ROM, PROM, EPROM, EEPROM, or any other suitable forms of memory known in the art. Memory member (1656) and/or controller (1660) is in electrical communication with main circuit board (1652) via flexible circuit (1654). Flexible circuit (1654) allows memory member (1656) to be placed separate and apart from main circuit board (1652). In some versions, memory member (1656) may be connected directly to main circuit board (1652) by soldering or with a plug and receptacle. Surgical instrument (1600) includes latch (1616) that is in electrical communication with memory member (1656) via a cable (1618). Latch (1616) is capable of transitioning from a closed position to an open position thereby transitioning first and second shroud portions (1612, 1614) from a connected state to a disconnected state. Additionally, latch (1616) in the open position engages a set of contacts that renders data stored on memory member (1656) unreadable, such as by electrically resetting, rewriting, or scrambling memory, by providing electrical communication with one of the follow reset elements: an integrated capacitor to supply voltage or current into memory member (1656), a current reverser to apply reverse current (i.e., reverse polarity), a power source capable of producing an electrical pulse that damages data stored on memory member (1656), and/or another memory (not shown) that releases unstable data (i.e., noise) to render memory member (1656) inoperable. When reverse polarity is used to destroy memory member (1656), a greater voltage than the voltage used in normal operation may be applied with low amperage to negative terminal or ground rather than positive terminal. For example, 10 volts supplied with low amperage is supplied to ground connections of a memory member (1656) that during normal operation uses 6 volts, although such configuration is merely one example and not intended to unnecessarily limit the invention.

FIG. 16 shows a circuit assembly (1750) for incorporation into any of surgical instrument (112, 152, 154, 156, 1000, 1100, 1200, 1400, 1500, 1600). Circuit assembly (1750) includes a main circuit board (1752), a memory member (1756), a controller (not shown), and a flexible circuit (1754). Memory member (1756), as mentioned above, may include a memory such as RAM, ROM, PROM, EPROM, EEPROM, or any other suitable forms of memory known in the art. Memory member (1756) is positioned within a body assembly (not shown) that houses, supports, and inhibits access to circuit assembly (1750). Flexible circuit (1754) includes a failure region (1762) that allows for removal of memory member (1756) and/or destruction of flexible circuit (1754) during the sterilization process. In the present version, failure region (1762) includes a conductive epoxy (1764) that bridges failure region (1762) to provide electrical communication between memory member (1556) and main circuit board (1752). When circuit assembly (1750) is heated to a temperature suitable to sterilize surgical instrument (1700), conductive epoxy (1764) destructs, such as by melting, in a manner that separates flexible circuit (1754) at failure region (1762) into one or more portions. Flexible circuit (1754) in a separated state disallows electrical communication between memory member (1756) and main circuit board (1752). In other versions, failure region (1762) is perforated or otherwise weakened so that a user may manually remove memory member (1756) from main circuit board (1752) with a hand or hands of the user. Additionally, controller may be attached to main circuit board (1752) with flexible circuit (1754) having failure region (1762) for ease of removal to be placed in a separate waste stream than main circuit board (1752). In other versions, controller may be similarly attached with a flexible circuit (1754) including a failure region (1762). Once removed, memory member (1756) and/or controller is inhibited from being used again in another circuit assembly.

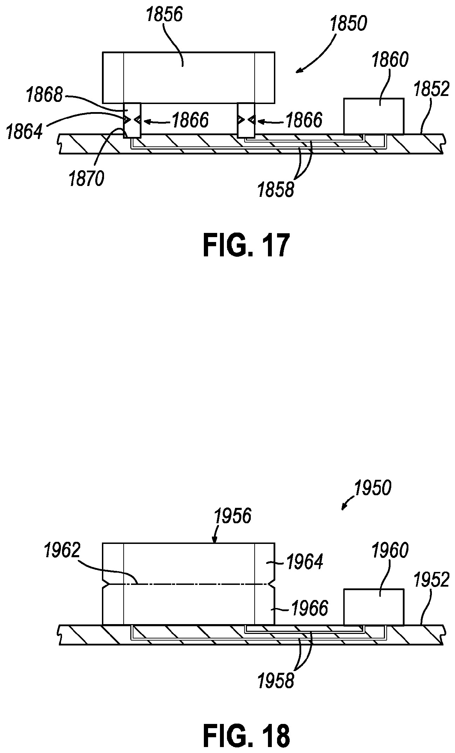

FIG. 17 shows a circuit assembly (1850) for incorporation into any of surgical instruments (112, 152, 154, 156, 1000, 1100, 1200, 1400, 1500, 1600). Circuit assembly (1850) is similar to circuit assembly (1750) except as otherwise described herein. Circuit assembly (1850) includes a main circuit board (1852), an integrated circuit (1858), a memory member (1856), and a controller (1860). Circuit assembly (1850) differs from circuit assembly (1750) in that memory member (1856) is attached to main circuit board (1852) by a pin connector (1868). Pin connector (1868) is a rigid connector constructed of metal capable of being soldered. Pin connector (1868) is directly soldered into a pin bore (1870). Pin bore (1870) is defined by main circuit board (1852). Pin connector (1868) includes a failure region (1866). Failure region (1866) allows for the removal of memory member (1856) manually prior to or during the sterilization process. As shown, failure region (1866) includes a conductive epoxy (1864) that bridges a gap in pin connector (1868). During the sterilization process, conductive epoxy (1864) reaches its predetermined melting temperature and derogates, such as by melting, when heated to a temperature suitable to sterilize a surgical instrument, such as any of surgical instruments (112, 152, 154, 156, 1000, 1100, 1200, 1400, 1500, 1600). Once conductive epoxy (1864) melts, two portions of pin connectors (1868) separate such that memory member (1856) from main circuit board (1852) disallows communication by integrated circuit (1858) to controller (1860).

In other versions, failure region (1866) includes a portion of pin connector (1868) that has a reduced diameter relative to a remaining portion of pin connector (1868). Failure region (1866) provides a location from which memory member (1856) may be broken away from main circuit board (1852) by a user's hand or hands with a reduced force (relative to the force to remove a memory member (1856) without a failure region (1866)) rendering the instrument inoperable and/or to provide for separate disposal in a separate waste stream than the other components of main circuit board (1852).

FIG. 18 shows a circuit assembly (1950) for incorporation into any of surgical instruments (112, 152, 54, 156, 1000, 1100, 1200, 1400, 1500, 1600). Circuit assembly (1950) is similar to circuit assembly (1850) except as otherwise described herein. Circuit assembly (1950) includes a main circuit board (1952), a memory member (1956), an integrated circuit (1958), and a controller (1960). Circuit assembly (1950) differs from circuit assembly (1850) in that main circuit board (1952), controller (1960) and/or memory member (1956) includes a frangible notch (1962) defining a weak section on main circuit board (1952), controller (1960), and/or memory member (1956). In the present version, memory member (1956) includes a first memory portion (1964), a second memory portion (1966), and frangible notch (1962). First memory portion (1964) is separated from second memory portion (1966) by frangible notch (1962). Second memory portion (1966) is rigidly attached to main circuit board (1952). First memory portion (1964) is removably coupled to second memory portion (1966) so that if main circuit board (1952) is removed from a body assembly (not shown), first memory portion (1964) is configured to engage a portion of body assembly and break memory member (1956) to thereby render memory member (1956) inoperable. In this respect, frangible notch (1962) is configured to inhibit memory member (1956) from being removed from main circuit board (1952) intact for reuse. Additionally, if a user attempts to install circuit assembly (1950) or inadvertently loaded circuit assembly (1950) into a sterilized or improper instrument, a portion of body assembly engages a first memory portion (1964) and damages memory member (1956) by separating first memory portion (1964) from second memory portion (1966) preventing reuse.

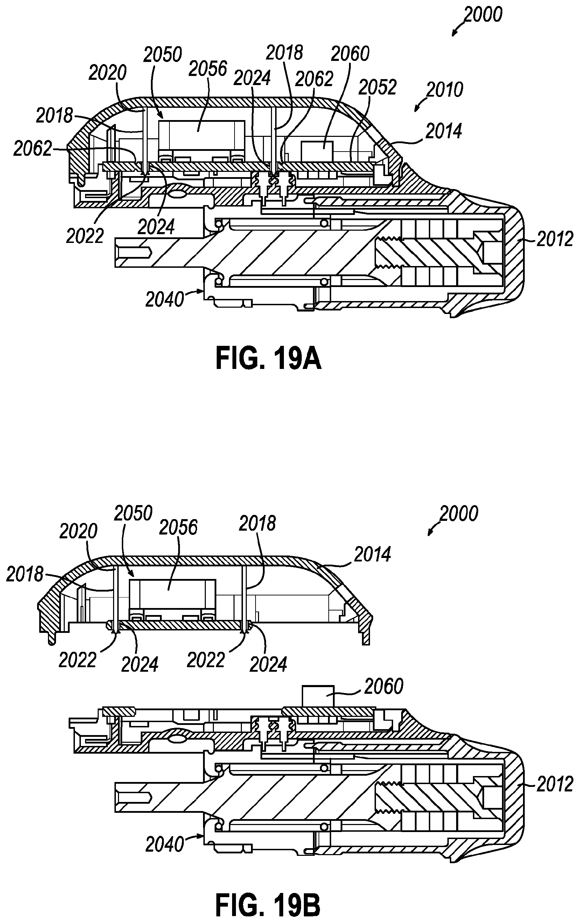

FIG. 19A shows a portion of surgical instrument (2000) similar to surgical instrument (1500) except as otherwise described herein. Body assembly (2010) includes a first shroud portion (2012), a second shroud portion (2014), and one or more support members (2018). First shroud portion (2012) provides support for a portion of energy drive system (2040) and a portion of circuit assembly (2050). Second shroud portion (2014) is affixed to first shroud portion (2012) in the connected state. Support members (2018) are rigid members that include a first support end (2020) and a second support end (2022). First support end (2020) is affixed to one of first shroud portion (2012) or second shroud portion (2014), and second support end (2022) attaches to circuit assembly (2050). Support members (2018) of the present example pass through a bore (2024) defined by circuit assembly (2050) and attach to the other of first shroud portion (2012) or second shroud portion (2014). Support members (2018) include a second support end (2022) that has a catch, such as a barb or a hook, configured for one-way installation into bore (2024). In this respect, the term "one-way installation" refers to catch being configured to install easily without damage, but to cause damage upon deinstallation. Circuit assembly (2050) includes a main circuit board (2052), a memory member (2056), and a controller (2060) and is shown in the operable state. Circuit assembly (2050) of the present example further includes a frangible separator (2062) proximate to support member (2018). Also in the present example, second shroud portion (2014) is configured to be transitioned to a disconnected state by moving second shroud portion (2014) relative to first shroud portion (2012), such as by moving second shroud portion (2014) horizontally. However, such movement is merely one exemplary direction and is not intended to unnecessarily limit the invention. By way of further example, second shroud portion (2014) may be removed vertically, slantwise, or another direction relative to first shroud portion (2012).

FIG. 19B shows portion of surgical instrument (2000) with second shroud portion (2014) removed from first shroud portion (2012). Support members (2018) move with second shroud portion (2014) away from first shroud portion (2012). Circuit assembly (2050) is held in place by first shroud portion (2012). Support members (2018) being transitioned away from first shroud portion (2012), cause damage to circuit assembly (2050) to render circuit assembly (2050) inoperable by breaking circuit assembly (2050) along frangible separators (2062), thus preventing reuse of circuit assembly (2050) and/or providing for placement in separate waste streams.

### IV. Exemplary Surgical Instrument Incorporating Selectively Separable Energy Drive System

In some instances, it may be desirable to provide a surgical instrument that includes components capable of delivering ultrasonic energy, RF energy, or both ultrasonic and RF energy. It may be desirable that these surgical instruments be easily openable so that the internal components may be separated into separate waste streams with minimal tools, such as no tools, within the sterile field. These surgical instruments are configured to stay intact during normal use but facilitate disassembly of internal components and/or selectively break the internal components. One such internal component is an energy drive system that is configured to be removed, separated by a hand or hands of the user, and placed into separate waste streams. These waste streams include but are not limited to recycling, disposal, or refurbishing.

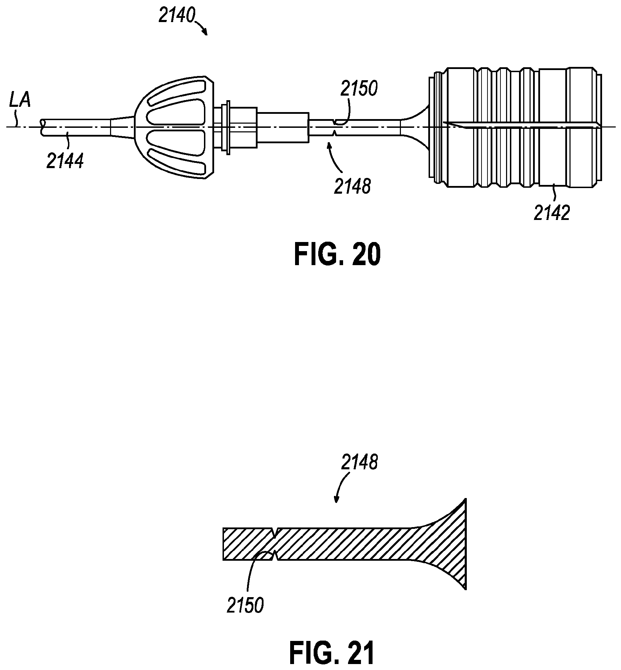

FIGS. 20-21 show a portion of an energy drive system (2140) for incorporation into any of surgical instruments (112, 152, 154, 156, 1000, 1100, 1200, 1400, 1500, 1600, 1700). Energy drive system (2140) includes an ultrasonic transducer (2142), a waveguide (2144), an energy coupling (2148), and an ultrasonic blade (not shown). Ultrasonic transducer (2142) extends along a longitudinal axis (LA), and energy coupling (2148) is configured to removably couple ultrasonic transducer (2142) to waveguide (2144). Waveguide (2144) further extends along longitudinal axis (LA) and is operatively affixed to ultrasonic blade. Energy coupling (2148) has a frangible division (2150) that is capable of being broken by a hand or hands of the user. Frangible division (2150) may include drilled holes, serrations, notches, or any other weakening for breaking in a predetermined manner. Once energy coupling (2148) is broken, ultrasonic transducer (2142) cannot be joined with waveguide (2144) without further reconditioning. By way of example, additional frangible divisions (2150) may be positioned between waveguide (2144) and ultrasonic blade. Ultrasonic transducer (2142) may thus be sanitized, refurbished, and reused in a similar surgical instrument while waveguide (2144) and ultrasonic blade are placed in another waste stream, such as disposal or recycling.

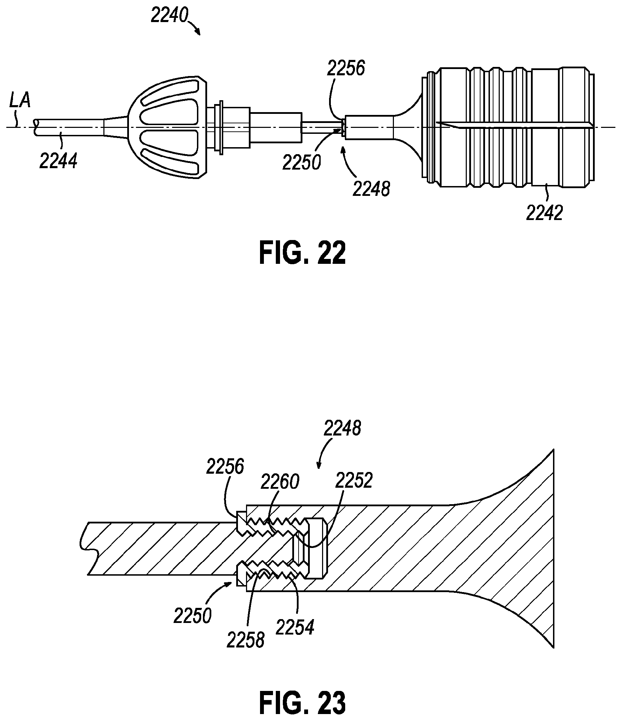

FIGS. 22-23 show another portion of an energy drive system (2240) for incorporation into any of surgical instruments (112, 152, 154, 156, 1000, 1100, 1200, 1400, 1500, 1600, 1700) similar to energy drive system (2140) except as otherwise described herein. Like energy drive system (2140), energy drive system (2240) includes an ultrasonic transducer (2242), a waveguide (2244), an energy coupling (2248), and an ultrasonic blade (not shown). Energy drive system (2240) differs from energy drive system (2140) in that energy drive system (2240) includes energy coupling (2248) configured to uncouple and recouple waveguide (2244) with ultrasonic transducer (2242). Energy coupling (2248) in the present example is in the form of a bushing (2250) configured to be threaded between ultrasonic transducer (2242) and waveguide (2244). Bushing (2250) includes an internal bushing thread (2252) and an external bushing thread (2254). Bushing (2250) also includes a torque feature (2256) in the form of a hex head configured to allow a user to tighten bushing (2250) within ultrasonic transducer (2242) to a specified torque, such as with a torque wrench (not shown) provided within a sterile packaging (not shown). As shown, external bushing thread (2254) is threaded into an internal transducer thread (2258), and external waveguide thread (2260) is threaded into internal bushing thread (2252), although this configuration may be reversed in other examples. Additionally, bushing (2250) may include two sets of internal threads, or two sets of external threads to be mated with complementary threads positioned on waveguide (2244) and ultrasonic transducer (2242). For example, external bushing threads (2254) threadedly couple with an internal waveguide thread (not shown) and an external transducer thread (not shown). Bushing (2250) is configured to transmit ultrasonic energy between ultrasonic transducer (2242) and waveguide (2244). Bushing (2250) of the present example is constructed of a less durable material than waveguide (2244) and/or ultrasonic transducer (2242) so that waveguide (2244) and/or ultrasonic transducer (2242) is preserved thereby eliminating the preference to refurbish waveguide (2244) and/or ultrasonic transducer (2242).

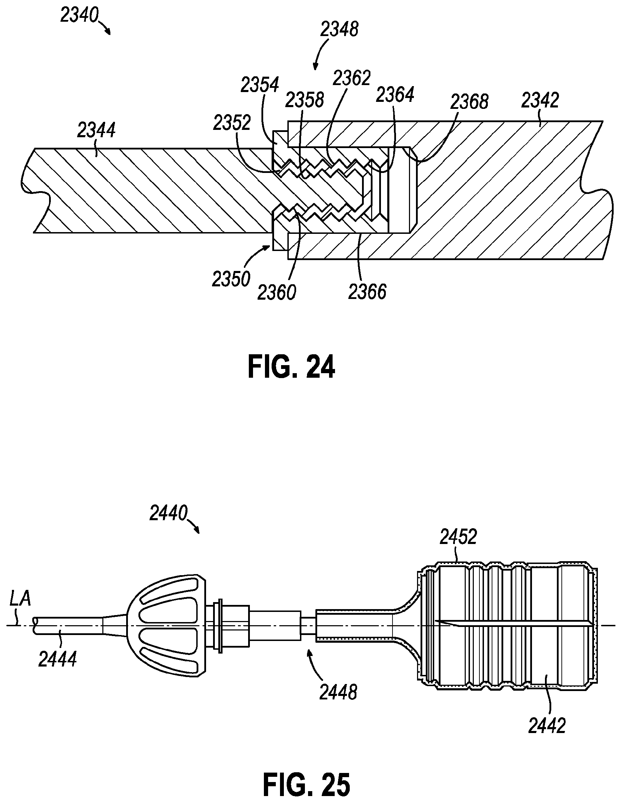

FIG. 24 shows a portion of an energy drive system (2340) including another energy coupling (2348) to be incorporated into any of surgical instruments (112, 152, 154, 156, 1000, 1100, 1200, 1400, 1500, 1600, 1700). Energy coupling (2348) is similar to energy coupling (2248) except as otherwise described herein. Energy coupling (2348), like energy coupling (2248), is capable of threadedly coupling a waveguide (2344) with an ultrasonic transducer (2342). Energy coupling (2348) more particularly includes a bushing assembly (2350) with a screw thread insert (2352) and a bushing (2354).

Waveguide (2344) includes a proximal end having an external waveguide thread (2360), and ultrasonic transducer (2343) including an internal transducer surface (2368). Screw thread insert (2352) of the present example is constructed of a helically wound wire, but may alternatively be a solid, machined piece. Screw thread insert (2352) includes an internal insert thread (2358) configured to engage an external waveguide thread (2360) and an external insert thread (2362) configured to engage internal bushing threads (2364). Bushing (2354) includes an internal bushing thread (2364) configured to be threaded around outer insert thread (2362). Bushing (2354) further includes an external bushing surface (2366) having a smooth bore sized for an interference fit relative to an internal transducer surface (2368). Bushing (2354) is press fit within ultrasonic transducer (2342). In some versions, external waveguide thread (2360) is mated with inner insert thread (2358), external insert thread (2362) is mated with internal bushing thread (2364), and external bushing surface (2366) is press fit within ultrasonic transducer (2342). Screw thread insert (2352) is separable from bushing (2354) so that, after use, ultrasonic transducer (2342) or waveguide (2344) may be removed for recycling or refurbishment and either screw thread insert (2352) and/or bushing (2350) may be disposed or recycled. Screw thread insert (2352) and bushing (2354) may be constructed of materials that conduct ultrasonic energy, but are less wear resistant than waveguide (2344) and/or ultrasonic transducer (2342) so screw thread insert (2352) and/or bushing (2354) may be replaced after a predetermined number of uses or after a predetermined amount of wear develops to prevent ultrasonic transducer (2342) and/or waveguide (2344) from developing wear.

FIG. 25 shows a portion of an energy drive system (2440) for incorporation into any of surgical instruments (112, 152, 154, 156, 1000, 1100, 1200, 1400, 1500, 1600, 1700). Energy drive system (2440) is similar to energy drive system (2240) except as otherwise described herein. Energy drive system (2440) includes an ultrasonic transducer (2442), a waveguide (2444), and an ultrasonic blade (not shown). Energy drive system (2440) may further include an energy coupling (2448) to further aid a user in disassembling energy drive system (2440) with a hand or hands of a user. Ultrasonic transducer (2442) extends along a longitudinal axis (LA) and may be removably coupled to waveguide (2444) by energy coupling (2448). Waveguide (2444) further extends along longitudinal axis (LA) and is operatively affixed to ultrasonic blade.

Ultrasonic transducer (2442) may be removed and refurbished for reuse. More particularly, ultrasonic transducer (2442) includes a protective coating, such as a cover (2452), to inhibit damage to ultrasonic transducer (2442) when removed from body assembly (2410). Cover (2452) includes a non-conductive base such as rubber, plastic, or ceramic. Cover (2452) of the present example also includes a conductive base installed by electroplating or by being topically applied to the surface of ultrasonic transducer (2442). Such protective coating may be applied as a liquid and allowed to dry. In other versions, cover (2452) is in a solid state and is fastened over ultrasonic transducer (2442).

### V. Exemplary Surgical Instrument Incorporating Selectively Separable Housing and Strain Relief Feature

In some instances, it may be desirable to provide a surgical instrument that includes components capable of delivering ultrasonic energy, RF energy, or both ultrasonic and RF energy that is easily opened so that the internal components may be separated into separate waste streams with minimal tools, such as no tools, within the sterile field. These surgical instruments are configured to stay intact during normal use but facilitate disassembly of internal components and/or selectively break the internal components. One such internal component is an electrical cable that is configured to be removed by a hand or hands of the user after separating a housing for access to the electrical cable. The electrical cable is configured to be placed into a waste stream that may include, but are not limited to, recycling, disposal, or refurbishing. Other components may be placed in waste streams other than the waste stream desired for electrical cable.

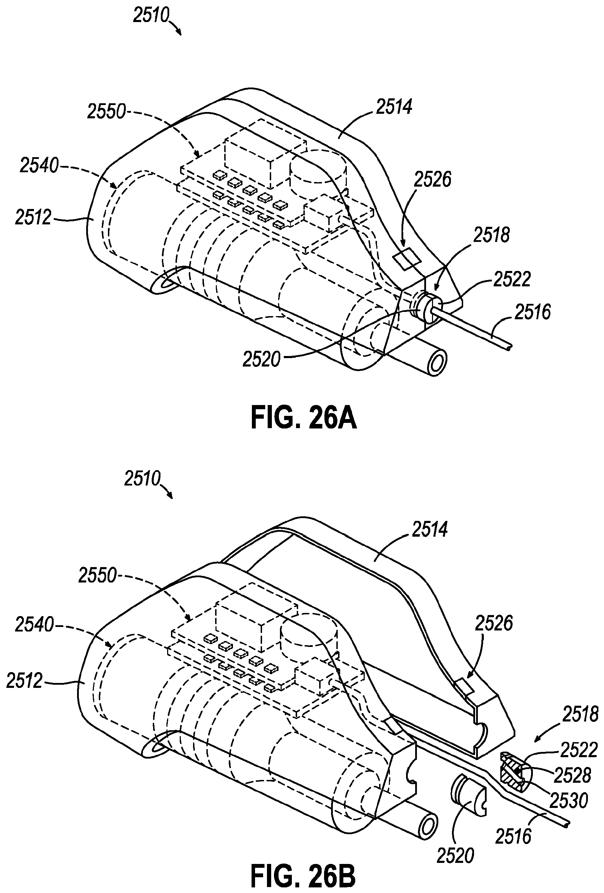

FIG. 26A shows a portion of a body assembly (2510) for incorporation into any of surgical instruments (112, 152, 154, 156, 1000, 1100, 1200, 1400, 1500, 1600, 1700). Body assembly (2510) includes a first shroud portion (2512), a second shroud portion (2514), a lateral coupling (2526), and an electrical cable (2516). First and second shroud portions (2512, 2514) more particularly are in the form of first and second cover portions. Body assembly (2510) is configured to support a circuit assembly (2550) and an energy drive system (2540) while also inhibiting access to circuit assembly (2550) and energy drive system (2540). Electrical cable (2516) provides electrical communication between a generator (150) (*see* FIG. 5) and energy drive system (2540) and/or circuit assembly (2550). With first shroud portion (2512) and second shroud portion (2514) retain electrical cable (2516) in a connected state. Lateral coupling (2526) is positioned between first and second shroud portions (2512, 2514) and is configured to retain first and second shroud portions (2512, 2514) in the connected state when positioned in a locked position. Lateral coupling (2526) may include any of the aforementioned shroud couplings (1004, 1204, 1206), magnetic lock assemblies (1134) and latches (1516, 1616) as previously discussed. Lateral coupling (2526) may be locked and unlocked with a key, electrically, or have magnetic members that are separated from one another. Body assembly (2510) further includes a strain relief assembly (2518) including a first relief portion (2520) and a second relief portion (2522). In the present version, strain relief assembly (2518) is proximate to lateral coupling (2526). Strain relief assembly (2518) may be operatively attached to first and second shroud portions (2512, 2514), or integrally formed with first and second shroud portions (2512, 2514), or a separate component installed within a bore (2524) positioned between first and second shroud portions (2512, 2514). As shown in the present example, strain relief assembly (2518) is a separate component. Strain relief assembly (2518) includes a recess configured to mate with bore (2524). Recess axially locates strain relief assembly (2518) within first and second shroud portions (2512, 2514). Strain relief assembly (2518) is configured to retain electrical cable (2516) when first shroud portion (2512) and second shroud portion (2514) are in the connected state. Strain relief assembly (2518) includes an angled surface (2528) positioned on the first relief portion (2520) with a complementary surface (2530) on the second relief portion (2522) configured to axially bind electrical cable (2516) therebetween.

FIG. 26B shows strain relief assembly (2518) in a disconnected state with first shroud portion (2512) laterally spaced apart from second shroud portion (2514). In order to transition body assembly from the connected state to the disconnected state, a user transitions lateral coupling (2526) from the locked state to an unlocked state. First shroud portion (2512) is laterally spaced away from second shroud portion (2514) thereby releasing electrical cable (2516) from strain relief assembly (2518). First relief portion (2520) is separated from second relief portion (2522) and fully releases electrical cable (2516) when first and second shroud portions (2512, 2514) are in the disconnected state. First shroud portion (2512) is operatively attached to first relief portion (2520) and separates from second shroud portion (2514) that is operatively attached to second relief portion (2522). Once first relief portion (2520) is spaced apart from second relief portion (2522), electrical cable (2516) is configured to be removed from body assembly (2510) with a hand or hands of the user with reduced manual force.

### VII. Miscellaneous

Versions of the devices described above may have application in conventional medical treatments and procedures conducted by a medical professional, as well as application in robotic-assisted medical treatments and procedures.

It should be understood that any of the versions of instruments described herein may include various other features in addition to or in lieu of those described above. By way of example only, any of the instruments described herein may also include one or more of the various features disclosed in any of the various references that are cited herein. It should also be understood that the teachings herein may be readily applied to any of the instruments described in any of the other references cited herein, such that the teachings herein may be readily combined with the teachings of any of the references cited herein in numerous ways. Other types of instruments into which the teachings herein may be incorporated will be apparent to those of ordinary skill in the art.

In addition to the foregoing, the teachings herein may be readily combined with the teachings of U.S. Pat. App. Pub. No. 2024/0006048 A1, entitled "Method of Reclaiming Portions of Surgical Instruments for Remanufacturing and Sustainability".

In addition to the foregoing, the teachings herein may be readily combined with the teachings of U.S. Pat. App. Pub. No. 2024/0006810 A1, entitled "Surgical Instrument with Removeable Cable and Associated Couplings".

In addition to the foregoing, the teachings herein may be readily combined with the teachings of U.S. Pat. App. Pub. No. 2004/0000476 A1, entitled "Surgical System and Methods of Assembly and Disassembly of Surgical Instrument".

In addition to the foregoing, the teachings herein may be readily combined with the teachings of U.S. Pat. App. Pub. No. 2004/0000526 A1, entitled "Robotic Surgical System with Removable Portion and Method of Disassembling Same".

In addition to the foregoing, the teachings herein may be readily combined with the teachings of U.S. Pat. App. Pub. No. 2024/0001416 A1, entitled "System for Determining Disposal of Surgical Instrument and Related methods".

In addition to the foregoing, the teachings herein may be readily combined with the teachings of U.S. Pat. App. Pub. No. 2024/0000491 A1, entitled "Reclamation Packaging for Surgical Instrument and Related Methods".

In addition to the foregoing, the teachings herein may be readily combined with the teachings of U.S. Pat. App. Pub. No. 2024/0000475 A1, entitled "Surgical Instrument with Various Alignment Features and Methods for Improved Disassembly and Assembly".

In addition to the foregoing, the teachings herein may be readily combined with the teachings of U.S. Pat. App. Pub. No. 2024/0003820 A1, entitled "Surgical System and Methods for Instrument Assessment and Cleaning".

It should also be understood that any ranges of values referred to herein should be read to include the upper and lower boundaries of such ranges. For instance, a range expressed as ranging "between approximately 1.0 inches (2.54cm) and approximately 1.5 inches (3.81cm)" should be read to include approximately 1.0 inches (2.54cm) and approximately 1.5 inches (3.81cm), in addition to including the values between those upper and lower boundaries.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention as defined by the claims. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument (1500; 1600), comprising:
(a) a shaft assembly (1020) extending along a longitudinal axis;
(b) an end effector (1030) distally extending from the shaft assembly (1020);
(c) an energy drive system (1540) operatively connected to the end effector (1030) and configured to apply a radio frequency (RF) energy or an ultrasonic energy to a tissue of a patient via the end effector;
(d) a circuit assembly (1550; 1650) operatively connected to the energy drive system (1540), wherein the circuit assembly (1550; 1650) includes a memory (1556; 1656); and
(e) a body assembly (1510; 1610) proximally extending from the shaft assembly (1020) and including:
(i) a first shroud portion (1512; 1612),
(ii) a second shroud portion (1514; 1614), and
(iii) a shroud coupling configured to removably affix the first shroud portion (1512; 1612) to the second shroud portion (1514; 1614) in a connected state, wherein the shroud coupling is further configured to detach the first shroud portion (1512; 1612) from the second shroud portion (1514; 1614) in a disconnected state,
wherein the first and second shroud portions (1512, 1514; 1612, 1614) in the connected state enclose and inhibit access to at least a portion of at least one of the circuit assembly (1550; 1650) or the energy drive system (1540; 1640) for containment therein,
wherein the first and second shroud portions (1512, 1514; 1612, 1614) in the disconnected state allow access to the at least the portion of at least one of the circuit assembly (1550; 1650) or the energy drive system (1540; 1640) for removal of the at least the portion of at least one of the circuit assembly (1550; 1650) or the energy drive system (1540; 1640) from the body assembly (1510; 1610), and
**characterized in that** either:
i) the shroud coupling includes a latch (1516) configured to selectively move from a secured position to an unsecured position upon respectively transitioning from the connected state to the disconnected state, and wherein the latch (1516) includes a magnet that is configured to use a magnetic field to cause a disruption of electrical signals or a magnetic pulse that damages or scrambles memory, such that the latch (1516) is configured to erase the memory (1556) while selectively moving from the secured position to the unsecured position, or
ii) the circuit assembly (1650) includes a latch (1616) capable of transitioning from a closed position to an open position thereby transitioning the first and second shroud portions (1612, 1614) from the connected state to the unconnected state, wherein the latch (1616) in the open position is configured to render the memory (1656) inoperable, wherein the latch (1616) renders the memory (1656) inoperable with a reset element selected from the group consisting of: an integrated circuit, an integrated capacitor, a current reverser, and a hall effect sensor.

2. The surgical instrument of claim 1, wherein the shroud coupling includes a push-pin (1260), wherein the push-pin (1260) includes a plurality of resilient ribs (1264) configured to be inserted through a first bore (1216) defined by the first shroud portion (1212) and a second bore (1218) defined by the second shroud portion (1214), wherein the plurality of resilient ribs (1264) expand after being inserted through the first and second bores (1216, 1218) to retain the first and second shroud portions (1212, 1214) in a connected state.

3. The surgical instrument of claim 1 or claim 2, wherein the shroud coupling includes a push-pin (1204), wherein the push-pin (1204) includes a shank (1206) configured to include a first outer diameter in an unexpanded state, wherein the first outer diameter of the shank (1206) is sized to fit within a pair of bores (1216, 1218) that extends through the first and second shroud portions (1212, 1214), and a pin (1234) configured to be fitted within the shank (1206) to transition the shank (1206) to an expanded state thereby connecting the first shroud portion (1212) to the second shroud portion(1214).

4. The surgical instrument of any one of claims 1 - 3, wherein the energy drive system, includes:
(i) a transducer (2242),
(ii) a waveguide (2244), and
(iii) an energy coupling (2248) configured to removably connect the transducer (2242) to the waveguide (2244), wherein the energy coupling (2248) is selected from the group consisting of: a frangible division (2150), a press fit bushing (2354), and a threaded bushing (2250).

5. The surgical instrument of any preceding claim, wherein the shroud coupling includes a magnetic fastener, wherein the magnetic fastener includes a magnetic coupling having a first magnetic member (1006) operatively secured to the first shroud portion (1012) and a second magnetic member (1008) operatively secured to the second shroud portion (1014), wherein the magnetic members (1006, 1008) are selected from the group consisting of: a rare earth magnet, a ferromagnetic metal, and a electromagnet, wherein the first magnetic member (1006) is operatively secured to the second magnetic member (1008) by magnetic attraction, wherein the first and second shroud portions (1012, 1014) are configured to transition from the connected state to the disconnected state by separating the first magnetic member (1006) from the second magnetic member (1008) by a user.

6. The surgical instrument of claim 5, wherein the shroud coupling includes an alignment feature (1028, 1032) configured to align the first shroud portion (1012) with the second shroud portion (1014) while being transitioned from the connected state to the disconnected state thereby aligning the first magnetic member (1006) with the second magnetic member (1008).

7. The surgical instrument of any preceding claim, wherein the shroud coupling includes a magnetic fastener, wherein the magnetic fastener includes a magnetic lock assembly (1134) configured to connect the first shroud portion (1112) to the second shroud portion (1114) in a connected state, wherein the magnetic lock assembly (1134) includes at least one magnetic member (1106, 1108) selected from the group consisting of: a rare earth magnet, a ferromagnetic metal, and a electromagnet, wherein the magnetic lock assembly (1134) uses magnetic attraction to render the first and second shroud portions (1112, 1114) in a locked state that disallows inadvertent movement of the first and second shroud portions (1112, 1114) from the connected state to the disconnected state.

8. The surgical instrument of claim 7, wherein the magnetic lock assembly (1134) includes a key (1136) configured to transition the magnetic lock assembly (1134) from the locked state to an unlocked state.

9. The surgical instrument of claim 7 or claim 8, wherein the at least one magnetic member includes a first magnetic member (1106) operatively connected to the first shroud portion (1112) and a second magnetic member (1108) operatively connected to the second shroud portion (1114), wherein one of the first magnetic member (1106) or the second magnetic member (1108) is magnetically attracted to the other of the first magnetic member (1106) or the second magnetic member (1108), wherein the first and second magnetic members (1106, 1108) have high magnetic attraction between each other in the locked state and the first and second magnetic members (1106, 1108) have low magnetic attraction between each other in the unlocked state.

10. The surgical instrument of any preceding claim, wherein the circuit assembly includes a main circuit board (1752), wherein the memory (1756) is connected to the main circuit board (1752) by a circuit coupling, and wherein the memory (1756) is configured to be permanently separated from the main circuit board (1752) at the circuit coupling, and wherein the circuit coupling is selected from the group consisting of: a failure region (1762), a reduced diameter (1866) of a pin connector (1868), or a frangible notch (1962).

11. The surgical instrument of any preceding claim, wherein the circuit assembly includes a main circuit board (1352) and a sub-board (1354), wherein the sub-board (1354) is connected to the main circuit board (1352) by a pluggable coupling (1362), and wherein the sub-board (1354) is configured to be separated from the main circuit board (1352) at the pluggable coupling (1362).

12. The surgical instrument of any preceding claim, wherein the circuit assembly includes a first circuit portion (1464), a second circuit portion (1466), and a frangible separator (1462), wherein the frangible separator (1462) connects the first circuit portion (1464) to the second circuit portion (1466) in an operable state, and wherein the frangible separator (1462) is configured to permanently separate the first circuit portion (1464) from the second circuit portion (1466) in an inoperable state.

13. The surgical instrument of any preceding claim, further comprising a cable (2516), wherein the cable (2516) is captured by the first and second shroud portions (2512, 2514) in the connected state, and wherein the cable (2516) is released from the first and second shroud portions (2512, 2514) in the disconnected state.

14. A method of disassembling the surgical instrument according to any preceding claim, the method comprising:
(a) removing a first shroud portion with a hand or hands of the user; and
(b) removing a portion of the circuit assembly.

15. The method of claim 14, further comprising:
(c) removing the portion of the circuit assembly by breaking the circuit assembly with a hand or the hands of a user.

## Patentansprüche

1. Chirurgisches Instrument (1500, 1600), umfassend:
(a) eine Wellenanordnung (1020), die sich entlang einer Längsachse erstreckt;
(b) einen Endeffektor (1030), der sich distal von der Wellenanordnung (1020) erstreckt;
(c) ein Energieantriebssystem (1540), das wirkmäßig mit dem Endeffektor (1030) verbunden und konfiguriert ist, um über den Endeffektor eine Hochfrequenzenergie (HF-Energie) oder eine Ultraschallenergie auf ein Gewebe eines Patienten anzulegen;
(d) eine Schaltungsanordnung (1550; 1650), die wirkmäßig mit dem Energieantriebssystem (1540) verbunden ist, wobei die Schaltungsanordnung (1550; 1650) einen Speicher (1556; 1656) einschließt; und
(e) eine Körperanordnung (1510; 1610), die sich von der Wellenanordnung (1020) proximal erstreckt und einschließt:
(i) einen ersten Mantelabschnitt (1512, 1612),
(ii) einen zweiten Mantelabschnitt (1514, 1614) und
(iii) eine Mantelkopplung, die konfiguriert ist, um den ersten Mantelabschnitt (1512; 1612) in einem verbundenen Zustand entfernbar an dem zweiten Mantelabschnitt (1514; 1614) anzubringen, wobei die Mantelkopplung ferner konfiguriert ist, um den ersten Mantelabschnitt (1512; 1612) in einem getrennten Zustand von dem zweiten Mantelabschnitt (1514; 1614) zu lösen,
wobei der erste und zweite Mantelabschnitt (1512, 1514; 1612, 1614) in dem verbundenen Zustand mindestens einen Abschnitt von mindestens einem von der Schaltungsanordnung (1550; 1650) oder dem Energieantriebssystem (1540; 1640) zur Aufnahme darin umgeben und den Zugang dazu verhindern,
wobei der erste und der zweite Mantelabschnitt (1512, 1514; 1612, 1614) im getrennten Zustand Zugang zu dem mindestens einen Abschnitt von mindestens einem von der Schaltungsanordnung (1550; 1650) oder dem Energieantriebssystem (1540; 1640) zum Entfernen des mindestens einen Abschnitts von mindestens einem von der Schaltungsanordnung (1550; 1650) oder dem Energieantriebssystem (1540; 1640) aus der Körperanordnung (1510; 1610) ermöglichen, und
**dadurch gekennzeichnet, dass** entweder:
i) die Mantelkopplung einen Riegel (1516) einschließt, der konfiguriert ist, um sich beim jeweiligen Übergehen von dem verbundenen Zustand in den getrennten Zustand selektiv von einer befestigten Position in eine unbefestigte Position zu bewegen, und wobei der Riegel (1516) einen Magnet einschließt, der konfiguriert ist, um ein Magnetfeld zu verwenden, um eine Störung elektrischer Signale oder einen magnetischen Impuls zu bewirken, der den Speicher beschädigt oder den Inhalt unlesbar macht, sodass der Riegel (1516) konfiguriert ist, um den Speicher (1556) zu löschen, während er sich selektiv von der befestigten Position in die unbefestigte Position bewegt, oder
ii) die Schaltungsanordnung (1650) einen Riegel (1616) einschließt, der in der Lage ist, von einer geschlossenen Position in eine offene Position überzugehen, wodurch der erste und der zweite Mantelabschnitt (1612, 1614) von dem verbundenen Zustand in den unverbundenen Zustand übergehen, wobei der Riegel (1616) in der offenen Position konfiguriert ist, um den Speicher (1656) funktionsunfähig zu machen, wobei der Riegel (1616) den Speicher (1656) mit einem Rückstellelement funktionsunfähig macht, das ausgewählt ist aus der Gruppe bestehend aus: einer integrierten Schaltung, einem integrierten Kondensator, einem Stromrichtungsumkehrer und einem Hall-Effekt-Sensor.

2. Chirurgisches Instrument nach Anspruch 1, wobei die Mantelkopplung einen Druckstift (1260) einschließt, wobei der Druckstift (1260) eine Vielzahl von elastischen Rippen (1264) einschließt, die konfiguriert sind, um durch eine erste Bohrung (1216), die durch den ersten Mantelabschnitt (1212) definiert ist, und eine zweite Bohrung (1218), die durch den zweiten Mantelabschnitt (1214) definiert ist, eingeführt zu werden, wobei sich die Vielzahl von elastischen Rippen (1264) nach dem Einführen durch die erste und zweite Bohrung (1216, 1218) ausdehnt, um den ersten und zweiten Mantelabschnitt (1212, 1214) in einem verbundenen Zustand zu halten.

3. Chirurgisches Instrument nach Anspruch 1 oder Anspruch 2, wobei die Mantelkopplung einen Druckstift (1204) einschließt, wobei der Druckstift (1204) einen Schaft (1206), der konfiguriert ist, um in einem nicht ausgedehnten Zustand einen ersten Außendurchmesser einzuschließen, wobei der erste Außendurchmesser des Schafts (1206) so bemessen ist, dass er in ein Paar von Bohrungen (1216, 1218) passt, die sich durch den ersten und zweiten Mantelabschnitt (1212, 1214) erstrecken, und einen Stift (1234) einschließt, der konfiguriert ist, um in den Schaft (1206) eingepasst zu werden, um den Schaft (1206) in einen ausgedehnten Zustand zu überführen, wodurch der erste Mantelabschnitt (1212) mit dem zweiten Mantelabschnitt (1214) verbunden wird.

4. Chirurgisches Instrument nach einem der Ansprüche 1 bis 3, wobei das Energieantriebssystem einschließt:
(i) einen Wandler (2242),
(ii) einen Wellenleiter (2244) und
(iii) eine Energiekopplung (2248), die konfiguriert ist, um den Wandler (2242) entfernbar mit dem Wellenleiter (2244) zu verbinden, wobei die Energiekopplung (2248) aus der Gruppe ausgewählt ist, bestehend aus: einer Sollbruchstelle (2150), einer Presspassungsbuchse (2354) und einer Gewindebuchse (2250).

5. Chirurgisches Instrument nach einem der vorstehenden Ansprüche, wobei die Mantelkopplung ein magnetisches Befestigungselement einschließt, wobei das magnetische Befestigungselement eine magnetische Kopplung einschließt, die ein erstes magnetisches Element (1006), das wirksam an dem ersten Mantelabschnitt (1012) befestigt ist, und ein zweites magnetisches Element (1008), das wirkmäßig an dem zweiten Mantelabschnitt (1014) befestigt ist, aufweist, wobei die magnetischen Elemente (1006, 1008) ausgewählt sind aus der Gruppe bestehend aus: einem Seltenerdmagneten, einem ferromagnetischen Metall und einem Elektromagneten, wobei das erste magnetische Element (1006) durch magnetische Anziehung wirkmäßig an dem zweiten magnetischen Element (1008) befestigt ist, wobei der erste und der zweite Mantelabschnitt (1012, 1014) konfiguriert sind, um durch Trennen des ersten magnetischen Elements (1006) von dem zweiten magnetischen Element (1008) durch einen Benutzer von dem verbundenen Zustand in den getrennten Zustand überzugehen.

6. Chirurgisches Instrument nach Anspruch 5, wobei die Mantelkopplung ein Ausrichtungsmerkmal (1028, 1032) einschließt, das konfiguriert ist, um den ersten Mantelabschnitt (1012) mit dem zweiten Mantelabschnitt (1014) auszurichten, während sie von dem verbundenen Zustand in den getrennten Zustand überführt werden, wodurch das erste magnetische Element (1006) mit dem zweiten magnetischen Element (1008) ausgerichtet wird.

7. Chirurgisches Instrument nach einem der vorstehenden Ansprüche, wobei die Mantelkopplung ein magnetisches Befestigungselement einschließt, wobei das magnetische Befestigungselement eine magnetische Verriegelungsanordnung (1134) einschließt, die konfiguriert ist, um den ersten Mantelabschnitt (1112) mit dem zweiten Mantelabschnitt (1114) in einem verbundenen Zustand zu verbinden, wobei die magnetische Verriegelungsanordnung (1134) mindestens ein magnetisches Element (1106, 1108) einschließt, ausgewählt aus der Gruppe bestehend aus: einem Seltenerdmagneten, einem ferromagnetischen Metall und einem Elektromagneten, wobei die magnetische Verriegelungsanordnung (1134) magnetische Anziehung nutzt, um den ersten und zweiten Mantelabschnitt (1112, 1114) in einen verriegelten Zustand zu versetzen, der eine unbeabsichtigte Bewegung des ersten und zweiten Mantelabschnitts (1112, 1114) aus dem verbundenen Zustand in den getrennten Zustand verhindert.

8. Chirurgisches Instrument nach Anspruch 7, wobei die magnetische Verriegelungsanordnung (1134) einen Schlüssel (1136) einschließt, der konfiguriert ist, um die magnetische Verriegelungsanordnung (1134) von dem verriegelten Zustand in einen entriegelten Zustand zu überführen.

9. Chirurgisches Instrument nach Anspruch 7 oder Anspruch 8, wobei das mindestens eine magnetische Element ein erstes magnetisches Element (1106), das wirkmäßig mit dem ersten Mantelabschnitt (1112) verbunden ist, und ein zweites magnetisches Element (1108), das wirkmäßig mit dem zweiten Mantelabschnitt (1114) verbunden ist, einschließt, wobei eines von dem ersten magnetischen Element (1106) oder dem zweiten magnetischen Element (1108) von dem anderen von dem ersten magnetischen Element (1106) oder dem zweiten magnetischen Element (1108) magnetisch angezogen wird, wobei das erste und das zweite magnetische Element (1106, 1108) im verriegelten Zustand eine hohe magnetische Anziehungskraft zueinander aufweisen und das erste und das zweite magnetische Element (1106, 1108) im entriegelten Zustand eine geringe magnetische Anziehungskraft zueinander aufweisen.

10. Chirurgisches Instrument nach einem der vorstehenden Ansprüche, wobei die Schaltungsanordnung eine Hauptleiterplatte (1752) einschließt, wobei der Speicher (1756) durch eine Schaltungskopplung mit der Hauptleiterplatte (1752) verbunden ist und wobei der Speicher (1756) konfiguriert ist, um an der Schaltungskopplung dauerhaft von der Hauptleiterplatte (1752) getrennt zu werden, und wobei die Schaltungskopplung ausgewählt ist aus der Gruppe bestehend aus: einem Versagensbereich (1762), einem reduzierten Durchmesser (1866) eines Stiftsteckverbinders (1868) oder einer Sollbruchkerbe (1962).

11. Chirurgisches Instrument nach einem der vorstehenden Ansprüche, wobei die Schaltungsanordnung eine Hauptleiterplatte (1352) und eine Nebenleiterplatte (1354) einschließt, wobei die Nebenleiterplatte (1354) durch eine steckbare Kopplung (1362) mit der Hauptleiterplatte (1352) verbunden ist und wobei die Nebenleiterplatte (1354) konfiguriert ist, um an der steckbaren Kopplung (1362) von der Hauptleiterplatte (1352) getrennt zu werden.

12. Chirurgisches Instrument nach einem der vorstehenden Ansprüche, wobei die Schaltungsanordnung einen ersten Schaltungsabschnitt (1464), einen zweiten Schaltungsabschnitt (1466) und ein Sollbruchtrennelement (1462) einschließt, wobei das Sollbruchtrennelement (1462) den ersten Schaltungsabschnitt (1464) mit dem zweiten Schaltungsabschnitt (1466) in einem betriebsfähigen Zustand verbindet und wobei das Sollbruchtrennelement (1462) konfiguriert ist, um den ersten Schaltungsabschnitt (1464) in einem nicht betriebsfähigen Zustand dauerhaft von dem zweiten Schaltungsabschnitt (1466) zu trennen.

13. Chirurgisches Instrument nach einem der vorstehenden Ansprüche, ferner umfassend ein Kabel (2516), wobei das Kabel (2516) in dem verbundenen Zustand durch den ersten und zweiten Mantelabschnitt (2512, 2514) gehalten wird und wobei das Kabel (2516) im getrennten Zustand von dem ersten und zweiten Mantelabschnitt (2512, 2514) freigegeben wird.

14. Verfahren zum Auseinandernehmen eines chirurgischen Instruments nach einem der vorstehenden Ansprüche, wobei das Verfahren umfasst:
(a) Entfernen eines ersten Mantelabschnitts mit einer Hand oder den Händen des Benutzers und
(b) Entfernen eines Abschnitts der Schaltungsanordnung.

15. Verfahren nach Anspruch 14, ferner umfassend:
(c) Entfernen des Abschnitts der Schaltungsanordnung durch Brechen der Schaltungsanordnung mit einer Hand oder den Händen eines Benutzers.

## Revendications

1. Instrument chirurgical (1500 ; 1600), comprenant :
(a) un ensemble tige (1020) s'étendant le long d'un axe longitudinal ;
(b) un effecteur terminal (1030) s'étendant de manière distale à partir de l'ensemble tige (1020) ;
(c) un système d'entraînement d'énergie (1540) connecté de manière fonctionnelle à l'effecteur terminal (1030) et conçu pour appliquer une énergie à radiofréquence (RF) ou une énergie ultrasonore à un tissu d'un patient par l'intermédiaire de l'effecteur terminal ;
(d) un ensemble de circuits (1550 ; 1650) connecté de manière fonctionnelle au système d'entraînement d'énergie (1540), dans lequel l'ensemble de circuits (1550 ; 1650) comporte une mémoire (1556 ; 1656) ; et
(e) un ensemble corps (1510 ; 1610) s'étendant proximalement à partir de l'ensemble tige (1020) et comportant :
(i) une première partie de protecteur (1512 ; 1612),
(ii) une seconde partie de protecteur (1514 ; 1614), et
(iii) un couplage de protecteur conçu pour fixer de manière amovible la première partie de protecteur (1512 ; 1612) à la seconde partie de protecteur (1514 ; 1614) dans un état connecté, dans lequel le couplage de protecteur est en outre conçu pour détacher la première partie de protecteur (1512 ; 1612) de la seconde partie de protecteur (1514 ; 1614) dans un état déconnecté,
dans lequel les première et seconde parties de protecteur (1512, 1514 ; 1612, 1614) à l'état connecté enferment et empêchent l'accès à au moins une partie d'au moins l'un parmi l'ensemble de circuits (1550 ; 1650) ou le système d'entraînement d'énergie (1540 ; 1640) pour un confinement dans celles-ci,
dans lequel les première et seconde parties de protecteur (1512, 1514 ; 1612, 1614) dans l'état déconnecté permettent l'accès à l'au moins une partie d'au moins l'un parmi l'ensemble de circuits (1550 ; 1650) ou le système d'entraînement d'énergie (1540 ; 1640) pour le retrait de l'au moins une partie d'au moins l'un parmi l'ensemble de circuits (1550 ; 1650) ou le système d'entraînement d'énergie (1540 ; 1640) de l'ensemble corps (1510 ; 1610), et
**caractérisé en ce que**, soit :
i) le couplage de protecteur comporte un verrou (1516) conçu pour passer sélectivement d'une position sécurisée à une position non sécurisée lors de la transition respective de l'état connecté à l'état déconnecté, et dans lequel le verrou (1516) comporte un aimant qui est conçu pour utiliser un champ magnétique pour provoquer une perturbation de signaux électriques ou une impulsion magnétique qui endommage ou brouille la mémoire, de telle sorte que le verrou (1516) est conçu pour effacer la mémoire (1556) tout en passant sélectivement de la position sécurisée à la position non sécurisée, soit
ii) l'ensemble de circuits (1650) comporte un verrou (1616) capable de passer d'une position fermée à une position ouverte, faisant de ce fait passer les première et seconde parties de protecteur (1612, 1614) de l'état connecté à l'état non connecté, dans lequel le verrou (1616) en position ouverte est conçu pour rendre la mémoire (1656) inutilisable, dans lequel le verrou (1616) rend la mémoire (1656) inutilisable avec un élément de réinitialisation choisi dans le groupe constitué de : un circuit intégré, un condensateur intégré, un inverseur de courant et un capteur à effet Hall.

2. Instrument chirurgical selon la revendication 1, dans lequel le couplage de protecteur comporte une punaise (1260), dans lequel la punaise (1260) comporte une pluralité de nervures élastiques (1264) conçues pour être insérées à travers un premier alésage (1216) défini par la première partie de protecteur (1212) et un second alésage (1218) défini par la seconde partie de protecteur (1214), dans lequel la pluralité de nervures élastiques (1264) se dilatent après avoir été insérées à travers les premier et second alésages (1216, 1218) pour retenir les première et seconde parties de protecteur (1212, 1214) dans un état connecté.

3. Instrument chirurgical selon la revendication 1 ou la revendication 2, dans lequel le couplage de protecteur comporte une punaise (1204), dans lequel la punaise (1204) comporte une queue (1206) conçue pour comporter un premier diamètre externe dans un état non expansé, dans lequel le premier diamètre externe de la queue (1206) est dimensionné pour rentrer à l'intérieur d'une paire d'alésages (1216, 1218) qui s'étend à travers les première et seconde parties de protecteur (1212, 1214), et une broche (1234) conçue pour rentrer à l'intérieur de la queue (1206) pour faire passer la queue (1206) à un état expansé, connectant ainsi la première partie de protecteur (1212) à la seconde partie de protecteur(1214).

4. Instrument chirurgical selon l'une quelconque des revendications 1 à 3, dans lequel le système d'entraînement d'énergie, comporte :
(i) un transducteur (2242),
(ii) un guide d'ondes (2244), et
(iii) un couplage d'énergie (2248) conçu pour relier de manière amovible le transducteur (2242) au guide d'ondes (2244), dans lequel le couplage d'énergie (2248) est choisi dans le groupe constitué par : une division frangible (2150), une douille à ajustement serré (2354) et une douille filetée (2250).

5. Instrument chirurgical selon l'une quelconque revendication précédente, dans lequel le couplage de protecteur comporte une fixation magnétique, dans lequel la fixation magnétique comporte un couplage magnétique ayant un premier élément magnétique (1006) sécurisé de manière fonctionnelle à la première partie de protecteur (1012) et un second élément magnétique (1008) fixé de manière fonctionnelle à la seconde partie de protecteur (1014), dans lequel les éléments magnétiques (1006, 1008) sont choisis dans le groupe constitué de : un aimant de terres rares, un métal ferromagnétique et un électroaimant, dans lequel le premier élément magnétique (1006) est fixé de manière fonctionnelle au second élément magnétique (1008) par attraction magnétique, dans lequel les première et seconde parties de protecteur (1012, 1014) sont conçues pour passer de l'état connecté à l'état déconnecté en séparant le premier élément magnétique (1006) du second élément magnétique (1008) par un utilisateur.

6. Instrument chirurgical selon la revendication 5, dans lequel le couplage de protecteur comporte une caractéristique d'alignement (1028, 1032) conçue pour aligner la première partie de protecteur (1012) avec la seconde partie de protecteur (1014) lors de la transition de l'état connecté à l'état déconnecté, alignant ainsi le premier élément magnétique (1006) avec le second élément magnétique (1008).

7. Instrument chirurgical selon l'une quelconque revendication précédente, dans lequel le couplage de protecteur comporte une fixation magnétique, dans lequel la fixation magnétique comporte un ensemble de verrouillage magnétique (1134) conçu pour connecter la première partie de protecteur (1112) à la seconde partie de protecteur (1114) dans un état connecté, dans lequel l'ensemble de verrouillage magnétique (1134) comporte au moins un élément magnétique (1106, 1108) choisi dans le groupe constitué : d'un aimant de terres rares, d'un métal ferromagnétique et d'un électroaimant, dans lequel l'ensemble de verrouillage magnétique (1134) utilise une attraction magnétique pour ramener les première et seconde parties de protecteur (1112, 1114) dans un état verrouillé qui empêche tout mouvement intempestif des première et seconde parties de protecteur (1112, 1114) de l'état connecté à l'état déconnecté.

8. Instrument chirurgical selon la revendication 7, dans lequel l'ensemble de verrouillage magnétique (1134) comporte une clé (1136) conçue pour faire passer l'ensemble de verrouillage magnétique (1134) de l'état verrouillé à un état déverrouillé.

9. Instrument chirurgical selon la revendication 7 ou la revendication 8, dans lequel l'au moins un élément magnétique comporte un premier élément magnétique (1106) relié de manière fonctionnelle à la première partie de protecteur (1112) et un second élément magnétique (1108) relié de manière fonctionnelle à la seconde partie de protecteur (1114), dans lequel l'un parmi le premier élément magnétique (1106) ou le second élément magnétique (1108) est attiré magnétiquement par l'autre parmi le premier élément magnétique (1106) ou le second élément magnétique (1108), dans lequel les premier et second éléments magnétiques (1106, 1108) ont une forte attraction magnétique entre eux à l'état verrouillé et les premier et second éléments magnétiques (1106, 1108) ont une faible attraction magnétique entre eux à l'état déverrouillé.

10. Instrument chirurgical selon l'une quelconque revendication précédente, dans lequel l'ensemble de circuits comporte une carte de circuit principale (1752), dans lequel la mémoire (1756) est connectée à la carte de circuit principale (1752) par un couplage de circuit, et dans lequel la mémoire (1756) est conçue pour être séparée de façon permanente de la carte de circuit principale (1752) au niveau du couplage de circuit, et dans lequel le couplage de circuit est choisi dans le groupe constitué de : une région de défaillance (1762), un diamètre réduit (1866) d'un connecteur à broche (1868) ou une encoche frangible (1962).

11. Instrument chirurgical selon l'une quelconque revendication précédente, dans lequel l'ensemble de circuits comporte une carte de circuit principale (1352) et une sous-carte (1354), dans lequel la sous-carte (1354) est connectée à la carte de circuit principale (1352) par un couplage enfichable (1362), et dans lequel la sous-carte (1354) est conçue pour être séparée de la carte de circuit principale (1352) au niveau du couplage enfichable (1362).

12. Instrument chirurgical selon l'une quelconque revendication précédente, dans lequel l'ensemble de circuits comporte une première partie de circuit (1464), une seconde partie de circuit (1466) et un séparateur frangible (1462), dans lequel le séparateur frangible (1462) connecte la première partie de circuit (1464) à la seconde partie de circuit (1466) dans un état fonctionnel, et dans lequel le séparateur frangible (1462) est conçu pour séparer de manière permanente la première partie de circuit (1464) de la seconde partie de circuit (1466) dans un état inutilisable.

13. Instrument chirurgical selon l'une quelconque revendication précédente, comprenant en outre un câble (2516), dans lequel le câble (2516) est capturé par les première et seconde parties de protecteur (2512, 2514) à l'état connecté, et dans lequel le câble (2516) est libéré des première et seconde parties de protecteur (2512, 2514) à l'état déconnecté.

14. Procédé de désassemblage de l'instrument chirurgical selon l'une quelconque revendication précédente, le procédé comprenant :
(a) le retrait d'une première partie de protecteur avec une main ou les mains de l'utilisateur ; et
(b) le retrait d'une partie de l'ensemble de circuits.

15. Procédé selon la revendication 14, comprenant en outre :
(c) le retrait de la partie de l'ensemble de circuits en brisant l'ensemble de circuits avec une main ou les mains d'un utilisateur.
